# EUROPEAN PATENT SPECIFICATION

(11) **EP 0 700 399 B1**
(45) Date of publication and mention of the grant of the patent: **04.10.2001**
(21) Application number: 94916937.9
(22) Date of filing: 05.05.1994
(51) Int. Cl.: C07D 498/08, C07D 213/80, A61K 31/44, A61K 31/395

(54) **36-DERIVATIVES OF RIFAMYCINS AND THEIR USE AS ANTIMICROBIAL AGENTS**
36-DERIVATE VON RIFAMYCINEN UND IHRE VERWENDUNG ALS ANTIMIKROBIELLES MITTEL
DERIVES-36 DE RIFAMYCINES ET LEUR UTILISATION COMME AGENTS ANTI-MICROBIENS

(30) Priority: 24.05.1993 EP 93108337; 29.06.1993 EP 93110315
(43) Date of publication of application: 13.03.1996
(73) Proprietor: GRUPPO LEPETIT S.P.A., 20020 Lainate (MI) (IT)
(72) Inventor: OCCELLI, Emilio, I-20015 Parabiago (IT); LOCIURO, Sergio, I-20143 Milano (IT); CIABATTI, Romeo, I-20026 Novate Milanese (IT); DENARO, Maurizio, Cincinnati, Ohio 45242 (US)
(74) Representative: Giambrocono, Alfonso, Dr. Ing.
(86) International application number: EP9401428
(87) International publication number: WO9428002

(56) References cited:
- HELVETICA CHIMICA ACTA vol. 56, no. 7 , 1973 , BASEL CH pages 2323 - 2347 W. KUMP ET AL 'Zur kenntnis von Rifamycin-S-Reaktionen des Ansaringes'
- JOURNAL OF ANTIBIOTICS. vol. 40, no. 12 , 1987 , TOKYO JP pages 1733 - 1739 W. WEHRLI ET AL 'CGP 4832, a new semisynthetic rifamycin derivative highly active against some Gram-negative bacteria'

## Description

This invention relates to rifamycin antibiotic derivatives of general formula I: 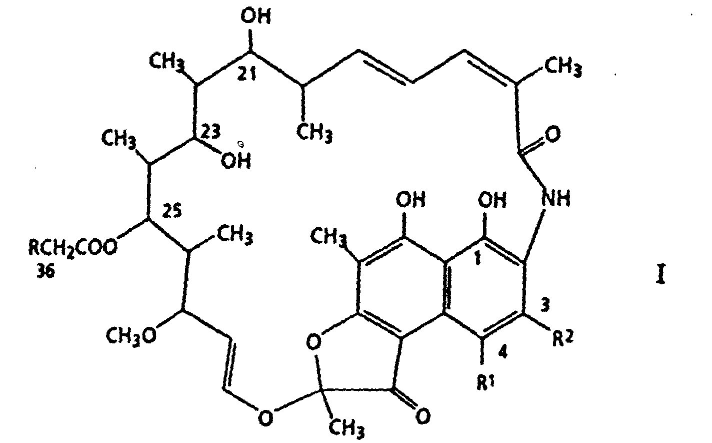 and to the oxidated derivatives thereof of formula Ia: 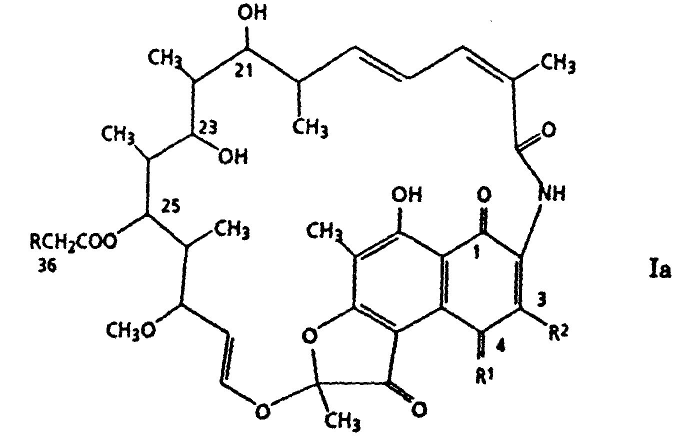 wherein:
R represents halo, hydroxy, thio, (C₁-C₄)alkoxy, (C₁-C₄)alkylthio, (C₁-C₄)acyloxy, (C₁-C₄)alkylamino, di(C₁-C₄)alkylamino or a group of formula: 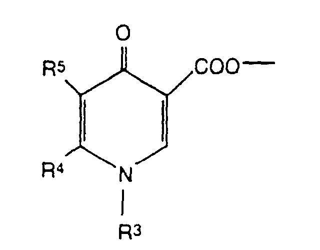 wherein:
   R³ represents (C₁-C₄)alkyl or (C₃-C₆)cycloalkyl;
   R⁴ represents a group of formula 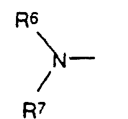 wherein:
      R⁶ and R⁷ independently represent hydrogen or (C₁-C₄)alkyl or
      R⁶ and R⁷ together with the adjacent nitrogen atom form a five or six membered heterocyclic ring, optionally containing one further heteroatom selected from oxygen, nitrogen and sulfur, wherein one of the carbon or nitrogen atoms of the ring is optionally substituted by a (C₁-C₄)alkyl moiety;
   R⁵ is hydrogen or halo;
   or R⁴ together with R⁵ form a bifunctional alkylenic chain, optionally containing 1 or 2 nitrogen atoms, of the following formula: 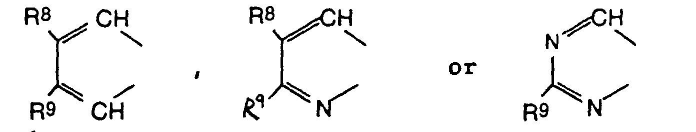 wherein:
      R⁸ represents hydrogen or halogen;
      R⁹ represents (C₁-C₄)alkyl, or a six membered heterocycle ring containing one or two nitrogen atoms, wherein the carbon and nitrogen atoms of the ring are optionally substituted with one or two (C₁-C₄)alkyl moieties;
   R¹ is hydroxy in formula I or oxygen in formula Ia;
   R² represents hydrogen, a five or six membered heterocyclic ring containing one or two heteroatoms selected from oxygen, nitrogen and sulfur, wherein one of the carbon or nitrogen atoms of the ring is optionally substituted by a (C₁-C₄)alkyl moiety, or a group of formula

      -CH=N-R¹⁰

      wherein R¹⁰ represents a six membered heterocycle ring containing one or two nitrogen atoms, wherein one of the carbon or nitrogen atoms of the ring is optionally substituted by (C₁-C₄)-alkyl or (C₅-C₆)cycloalkyl;
      or R¹ and R² taken together form a group of formula

      =N-(CHR¹¹)-X-, -NH-(CHR¹¹)-X-, or -N=(CR¹¹)-X-,

      wherein:
      X represents a sulfur atom or a -NH- group and
      R¹¹ represents hydrogen, (C₁-C₄)alkyl, (C₁-C₄)alkylamino or di(C₁-C₄)alkylamino;
and the pharmaceutically acceptale base addition salts thereof.

In the present description, the terms used above in defining the meanings of the substituents R¹ to R¹⁴ are intended to have the meanings commonly assigned to them in the art. Accordingly:
(C₁-C₄)alkyl represent a linear or branched hydrocarbon moiety containing 1 to 4 carbon atoms, such as:
   -CH₃,
   -CH₂-CH₃,
   -CH₂-CH₂-CH₃,
   -CH(CH₃)₂,
   -CH₂-CH₂-CH₂-CH₃,
   -CH(CH₃)-CH₂-CH₃,
   -CH₂-CH(CH₃)-CH₃,
   -C(CH₃)₃
   halo represents fluoro, chloro, bromo or iodo;
(C₁-C₄) alkoxy represents a linear or branched ether moiety containing 1, 2, 3 or 4 carbon atoms, such as:
   -O-CH₃,
   -O-CH₂-CH₃,
   -O-CH₂-CH₂-CH₃,
   -O-CH(CH₃)₂,
   -O-CH₂-CH₂-CH₂-CH₃,
   -O-CH(CH₃)-CH₂-CH₃,
   -O-CH₂-CH(CH₃)-CH₃,
   -O-C(CH₃)₃;
(C₁-C₄)acyloxy represents a carboxylic moiety containing 1 to 4 carbon atoms, such as:
   -O-CO-H
   -O-CO-CH₃,
   -O-CO-CH₂-CH₃,
   -O-CO-CH₂-CH₂-CH₃,
   -O-CO-CH(CH₃)₂;
(C₁-C₄)alkylthio represents a linear or branched thioether moiety with 1 to 4 carbon atoms, such as:
   -S-CH₃,
   -S-CH₂-CH₃,
   -S-CH₂-CH₂-CH₃,
   -S-CH(CH₃)₂,
   -S-CH₂-CH₂-CH₂-CH₃,
   -S-CH(CH₃)-CH₂-CH₃,
   -S-CH₂-CH(CH₃)-CH₃,
   -S-C(CH₃)₃;
(C₁-C₄)alkylamino represents an amino moiety substituted with a linear or branched alkyl containing 1 to 4 carbon atoms, such as:
   -NH-CH₃,
   -NH-CH₂-CH₃,
   -NH-CH₂-CH₂-CH₃,
   -NH-CH(CH₃)₂,
   -NH-CH₂-CH₂-CH₂-CH₃,
   -NH-CH(CH₃)-CH₂-CH₃,
   -NH-CH₂-CH(CH₃)-CH₃,
   -NH-C(CH₃)₃.
(C₁-C₄) dialkylamino represents an amino moiety substituted with two linear or branched alkyl moieties containing 1 to 4 carbon atoms such as:
   -N(CH₃)₂,
   -N(CH₃)-CH₂-CH₃,
   -N(CH₂-CH₃)₂,
   -N(CH₃)-CH₂-CH₂-CH₃,
   -N(CH₂-CH₃)-CH₂-CH₂-CH₃,
   -N(CH₂-CH₂-CH₃)₂,
   -N(CH₃)-CH(CH₃)₂,
   -N(CH₂-CH₃)-CH(CH₃)₂,
   -N(CH₃)-CH₂-CH₂-CH₂-CH₃,
   -N(CH₂-CH₃)-CH₂-CH₂-CH₂-CH₃,
   -N(CH₂-CH₂-CH₃)-CH₂-CH₂-CH₂-CH₃,
   -N(CH₂-CH₂-CH₂-CH₃)₂,
   -N(CH₂-CH₂-CH₂-CH₃)-CH(CH₃)₂.
(C₃-C₆)cycloalkyl represents a cyclic hydrocarbon moiety containing from 3 to 6 carbon atoms such as:
   cyclopropyl,
   cyclobutyl,
   cyclopentyl,
   cyclohexyl.

A five or six membered heterocyclic ring containing one or two heteroatoms selected from oxygen, nitrogen and sulfur (according to the definition of substituents R⁶ and R⁷ or R⁹) is an heterocyclic ring such as: 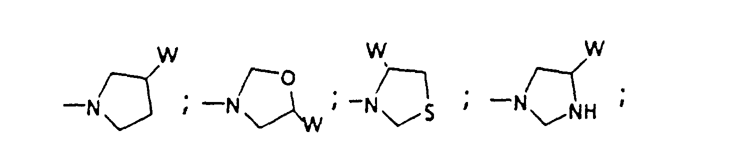 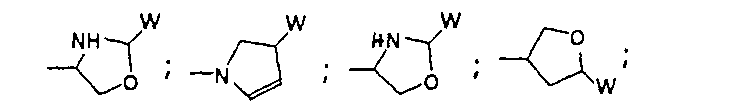 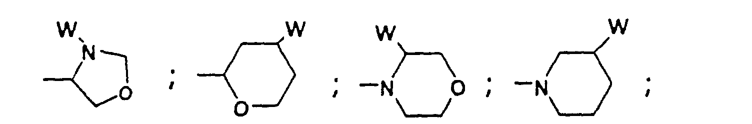 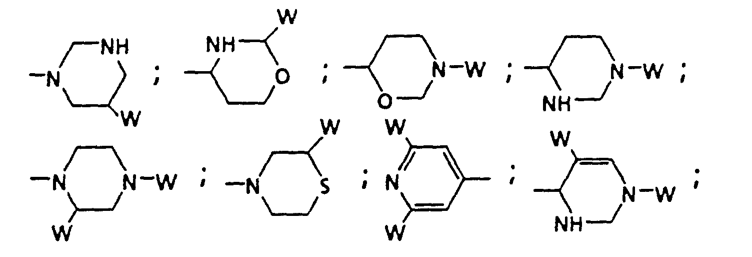 wherein W represents hydrogen or the various possible substituents of the 5 or 6 membered heterocyclic ring, according to the definitions set out above;

A bifunctional alkylenic chain, optionally containing 1 or 2 nitrogen atoms, according to the meanings of R² and R³ taken together, is a group which forms, with the two adjacent carbon atoms, a six membered aromatic heterocyclic ring, such as: 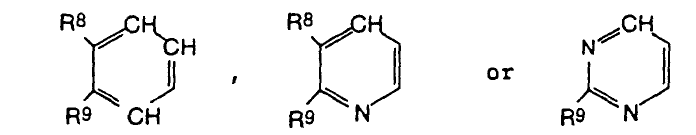 wherein the substituents R⁸ and R⁹ have the same meanings as in formula I;
A group of formula =N-(CHR¹¹)-X-, -NH-(CHR¹¹)-X- or -N=(CR¹¹)-X-, is a group which forms, with the adjacent carbon atoms in position 3 and 4, an heterocyclic ring such as: 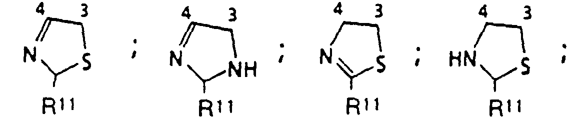 wherein R¹¹ is as above defined; obviously, the double bond between the nitrogen atom and the carbon atom in position 4 is only possible when the rifamycin is in the oxidated form.

Di(C₁-C₄)alkylamino represents an amino moiety substituted with two linear or branched alkyl groups containing 1, 2, 3 or 4 carbon atoms such as:
-N(CH₃)₂,
-N(CH₃)(CH₂-CH₃),
-N(CH₂-CH₃)₂,
-N(CH₃)(CH₂-CH₂-CH₃),
-N(CH₂-CH₃)(CH₂-CH₂-CH₃),
-N(CH₂-CH₂-CH₃)₂,
-N(CH₃)[CH(CH₃)₂],
-N(CH₂-CH₃)[CH(CH₃)₂],
-N(CH₃)(CH₂-CH₂-CH₂-CH₃),
-N(CH₂-CH₃)(CH₂-CH₂-CH₂-CH₃),
-N(CH₂-CH₂-CH₃)(CH₂-CH₂-CH₂-CH₃),
-N(CH₂-CH₂-CH₂-CH₃)₂,
-N(CH₂-CH₂-CH₂-CH₃)[CH(CH₃)₂].

Pharmaceutically acceptable base addition salts of the compounds of formula I are the rifamycin salts formed with alkali metal, earth-alkali metal, (C₁-C₄)alkylamines, (C₁-C₄)alkanolamines or basic aminoacids.

As known in the art, the hydroxy groups linked in positions 1 and 4 on the naphthalenic ring of the compound of formula I, may be both in the reduced form (in such case R¹ is hydroxy) or oxidated form (R¹ is oxo in this case).

These compounds are derivatives of rifamycin SV and of rifamycin S, respectively. The conversion from one form of rifamycin to the other, and viceversa, is easily carried out by means of oxidating or reducing reactions well known in the art; for instance the oxidation reaction may be carried out with manganese dioxide or potassium hexacyanoferrate(III) in chloroform, while the reducing reaction with ascorbic acid or sodium ascorbate in an hydroalcoholic solution.

The hydroxy moiety in position 1 is also in the oxidated form when the substituent R¹ together with R² (in the compound of formula I) form a group of formula =N-(CHR¹¹)-X-, wherein X and R¹¹ are as above defined; in this case the formation of the oxo moiety in 1 is a consequence of the double bonding between the nitrogen atom and the carbon atom in position 4.

Therefore, when particular substituents are not present, which would prevent the conversion from the reduced form into the oxidated one, or viceversa, in the continuing of this specification the derivatives of rifamycin S are to be considered as being convertible in the SV form and viceversa.

In the following specification, the term "rifamycins" is intended to comprise within its meanings all the suitable rifamycin and rifamycin-like compounds known in the art, such as rifamycin S, SV, P, the 3- and/or 4-derivatives thereof, and the pharmaceutically acceptable salts thereof.

The rifamycin antibiotic compounds are well known in the art, as being widely used for long time in the treatment of infections caused by Mycobacteria and Gram positive microorganisms and prophylaxis for certain Gram negative infections. The best known member of this antibiotic family is Rifampicin, which is one of the antibiotics of choice in the treatment of tuberculosis, whose main causative agent is Mycobacterium tuberculosis.

Rifamycin SV corresponds to the compound of formula I wherein the substituent R at position 36 is replaced by a hydrogen atom, R² is hydrogen and R¹ is hydroxy; rifamycin S is the oxidated form of rifamycin SV, as stated above; rifamycin P is the 4-desoxy-thiazolo-[5,4-c] rifamycin S; rifampicin is the 3-{[(4-methyl-1-piperazinyl)imino]-methyl} rifamycin SV.

The production of rifamycin SV may be obtained by fermenting variant cultures of the strain ATCC 13685 Nocardia mediterranei (previously named Streptomyces mediterranei, now renamed as Amycolatopsis mediterranei); for instance from Nocardia mediterranei ATCC 21271, as described in "The Journal of Antibiotics vol. 22, 12, 637, (1969)".

US 3884763 discloses a process for preparing rifamycin SV or rifamycin S by aerobically fermenting an aqueous nutrient medium containing a strain of Micromonospora chalcea ATCC 21994.

Rifamycin S and SV may also be obtained by chemical modification of the rifamycin B as described in US patent No. 3301753. Rifamycin B was first obtained as a component of a rifamycin complex by fermenting the Streptomyces mediterranei strain ATCC 13685 as described in US patent No. 3150046; rifamycin B may also be obtained as a single component by adding sodium ethyl barbiturate to the culture medium of Streptomyces mediterranei strain ATCC 13685, as described in US Patent 2988490 or by fermenting a variant culture of the strain ATCC 13685, i.e. the strain ATCC 21796, as described in US Patent 3871965.

Rifamycin P may be obtained either by fermentation of Streptomyces mediterranei ATCC 31064, ATCC 31065, ATCC 31066, as disclosed in US patent 4263404, or by chemical modification of rifamycin S, as described in US patent 4144234.

US patent 4880789 discloses the preparation of the 2'-N,N-dialkylamino derivatives of rifamycin P, by treating rifamycin P with dialkylamine in ethyl acetate; such derivatives may also be obtained by treating the 3-bromorifamycin S with N,N-dialkylthiourea, as described by "Cavalleri B. et al., J. of Med. Chem., 1990, 33, 1470-1476".

Rifampicin, which is a rifamycin SV bearing a [(4-methyl-1-piperazinyl)-imino]methyl group in position 3, may be obtained by reacting rifamycin SV with N-methylene-t-butylamine (obtained by reacting formaldehyde with t-butylamine) in the presence of manganese dioxide and then with 1-amino-4-methyl piperazine, as described in US patent 3542762.

Pharmaceutically acceptable salts of rifamycins are also well known in the art, and are easily obtainable by contacting the unsalified rifamycin derivative with the desired base.

For instance, US patent 3301753 discloses the alkali and earth-alkali metal salts of rifamycin SV; US Patent 4312866 discloses the preparation of rifamycin SV salts with basic aminoacids, such as arginine, lysine and histidine.

The reaction of 25-O-Deacetyl-3-morpholino rifamycin S-21,23-acetonide with malonic acid was described by W. Wehrli et al. (Journal of Antibiotics, Tokyo, 1987; 40, 1733). The obtained 25-O-Deacetyl-25-O-malonic acid-3-morpholino rifamycin S-21,23-acetonide was further reacted with hydroxybenzotriazole in THF and then with 3-hydroxymethyl-1-methylpiperidine in dicyclohexylcarbodiimmide, thus obtaining the 36-[[(1-methyl-3-piperidinyl)methoxy]carbonyl]-3-morpholino rifamycin S.

*25-O-deacetyl-25-O-propionyl and 25-O-deacetyl-25-O-pivaloyl derivatives of rifamycin are described by Kump W. et al., Helv. Chem. Acta, 1973, 56, 2323.*

It is known in the art that prolongated and extensive use of an antibiotic substance in the treatment of infections has the general drawback of the development of mutant strains of the pathogenic microorganism, which strains may show resistance against the specific antibiotic. It would therefore be desirable to produce new antibiotic substances, which are active both against the main causative agent, as well as against the mutant strains thereof which have developed resistance against the original antibiotic.

With the present invention, new antibiotic derivatives of rifamycin are provided which are mainly active against gram positive bacteria and gram positive as well as gram negative anaerobes, and show antimicrobial activity against rifamycin resistant strains.

Preferred compounds of formula I are those compounds wherein:
R is halo, hydroxy, (C₁-C₄)acyloxy, (C₁-C₄)alkoxy, (C₁-C₄)alkylthio, di(C₁-C₄)alkylamino or a group of formula: 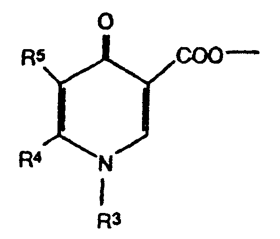 wherein:
   R³ represents (C₁-C₄)alkyl or (C₃-C₆)cycloalkyl;
   R⁴ represents a group of formula 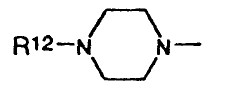 wherein R¹² represents hydrogen or (C₁-C₄)alkyl;
   R⁵ is hydrogen or halo;
   or R⁴ together with R⁵ form a bifunctional alkylenic chain, optionally containing 1 or 2 nitrogen atoms, of the following formula: 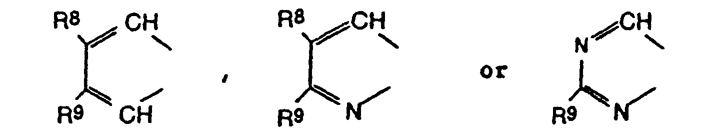 wherein R⁹ represents (C₁-C₄)alkyl, a group of formula 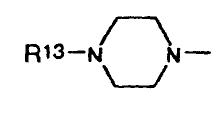 wherein R¹³ is hydrogen or (C₁-C₄)alkyl, or a group of formula 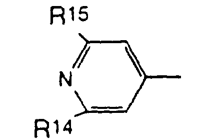 wherein R¹⁴ and R¹⁵ independently represent hydrogen or (C₁-C₄)alkyl;
R¹ is hydroxy in the reduced form or oxygen in the oxidated form;
R² represents hydrogen, a six membered heterocyclic ring containing one or two heteroatoms selected from oxygen, nitrogen and sulfur, wherein one of the carbon or nitrogen atoms of the ring is optionally substituted by a (C₁-C₄)alkyl moiety, or a group of formula: 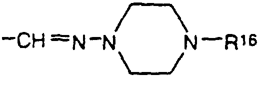 wherein R¹⁶ represents (C₁-C₄)alkyl or (C₅-C₆)cycloalkyl;
or R¹ and R² taken together form a group of formula -N=CR¹¹-S- wherein R¹¹ represents hydrogen, (C₁-C₄)alkyl or di(C₁-C₄)alkylamino.

Among the above compounds, further preferred compounds are those of formula I wherein:
R is fluoro, bromo, chloro, iodo, hydroxy, formyloxy, acetyloxy, thiomethyl diethylamino or a group of formula: 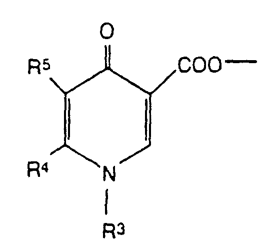 wherein:
R³ is ethyl or cyclopropyl, R⁴ is 4-methyl-1-piperazinyl and R⁵ is hydrogen;
or R⁴ together with R⁵ form a bifunctional alkylenic chain, optionally containing 1 or 2 nitrogen atoms, of formula: 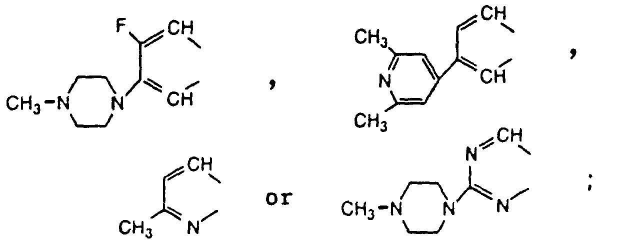
R¹ is hydroxy in the reduced form or oxygen in the oxidated form;
R² is hydrogen, 4-morpholinyl, {[(4-methyl-1-piperazinyl)imino]methyl} or {[(4-cyclopentyl-1-piperazinyl)imino]methyl};
or R¹ and R² together form a group of formula

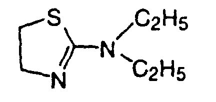

Particularly preferred compounds are those compounds of formula I wherein:
R is bromo, chloro, iodo, hydroxy or a group of formula: 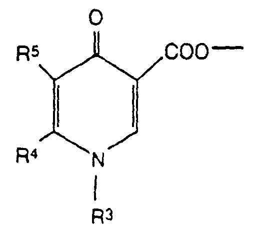 wherein:
R³ is ethyl, R⁴ is 4-methyl-1-piperazinyl and R⁵ is hydrogen;
or R⁴ together with R⁵ form a bifunctional alkylenic chain of formula: 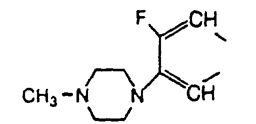
R¹ is hydroxy in the reduced form or oxygen in the oxidated form and R² is hydrogen, 4-morpholinyl or {[(4-methyl-1-piperazinyl)-imino]methyl}.

Representative examples of the compounds of the invention are:
36-bromorifamycin S
36-fluororifamycin S
36-chlororifamycin S
36-iodorifamycin S
36-methylthiorifamycin S
36-ethylthiorifamycin S
36-hydroxyrifamycin S
36-methoxyrifamycin S
36-ethoxyrifamycin S
36-formyloxyrifamycin S
36-acetyloxyrifamycin S
36-diethylaminorifamycin S
3,36-dibromorifamycin S
3,36-dichlororifamycin S
36-bromo-3-cyanorifaaycin S
36-hydroxy-3-cyanorifamycin S
36-methylthio-3-cyanorifamycin S
36-chloro-3-methylrifamycin S
36-bromo-3-ethylrifamycin S
36-acetyloxy-3-ethoxyrifamycin S
36-chloro-3-butoxyrifamycin S
36-bromo-3-methylthiorifamycin S
36-methylthio-3-ethoxycarbonylrifamycin S
36-chloro-3-(ethylpropylamino)rifamycin S
36-bromo-3-(diethylamino)rifamycin S
36-chloro-3-cyanorifamycin S
36-methylthio-3-(dimethylaminomethylene)rifamycin S
36-bromo-3-(ethylmethylaminomethylene)rifamycin S
36-bromo-3-(4-morpholinyl)rifamycin S
36-bromo-3-(4-(2-ethyl)-morpholinyl)rifamycin S
36-chloro-3-(4-(2-ethyl)-morpholinyl)rifamycin S
36-bromo-3-(1-piperidyl)rifamycin S
36-bromo-3-(3-(1-methyl)piperidyl)rifamycin S
36-iodo-3-(1-(3-methyl)-piperidyl)rifamycin S
36-chloro-3-(1-(3-methyl)-piperidyl)rifamycin S
36-acetyloxy-3-(1-piperazinyl)rifamycin S
36-bromo-3-(1-(3-methyl)piperazinyl)rifamycin S
36-hydroxy-3-(1-(3-methyl)piperazinyl)rifamycin S
36-fluoro-3-(4-morpholinyl)rifamycin S
36-chloro-3-(4-morpholinyl)rifamycin S
36-methylthio-3-(4-morpholinyl)rifamycin S
36-hydrox-3-(4-morpholinyl)rifamycin S
36-formyloxy-3-(4-morpholinyl)rifamycin S
36-acetyloxy-3-(4-morpholinyl)rifamycin S
36-diethylamino-3-(4-morpholinyl)rifamycin S
36-bromo-3-(4-thiomorpholinyl)rifamycin S
36-iodo-3-(4-thiomorpholinyl)rifamycin S
36-bromo-3-(4-(3-ethyl)thiomorpholinyl)rifamycin S
36-fluoro-3-(3-thiomorpholinyl)rifamycin S
36-chloro-3-(2-thiomorpholinyl)rifamycin S
36-methylthio-3-(4-thiomorpholinyl)rifamycin S
36-formyloxy-3-(4-thiomorpholinyl)rifamycin S
36-bromorifamycin P
36-fluororifamycin P
36-chlororifamycin P
36-iodorifamycin P
36-hydroxyrifamycin P
36-methy[thiorifamycin P
36-formyloxyrifamycin P
36-acetyloxyrifamycin P
36-diethylaminorifamycin P
36-bromo-2'-(methyl)rifamycin P
36-fluoro-2'-(diethylamino)rifamycin P
36-chloro-2'-(diethylamino)rifamycin P
36-methylthio-2'-(diethylamino)rifamycin P
36-formyloxy-2'-(diethylamino)rifamycin P
36-bromo-2'-(diethylamino)rifamycin P
36-hydroxy-2'-ethylrifamycin P
36-chloro-2'-(ethylamino)rifamycin P
36-bromo-2'-butylrifamycin P
36-acetyloxy-2'-(butylamino)rifamycin P
36-bromo-2'-(ethylmethylamino)rifamycin P
36-chloro-2'-ethylrifamycin P
36-[(1-ethyl-6-fluoro-1,4-dihydro-7-(4-methyl-1-piperazinyl)-4-oxo-3-quinolinyl)carbonyloxy] rifamycin S
36-{[1-ethyl-1,4-dihydro-7-methyl-4-oxo-1,8-naphthyridin-3-yl)carbonyloxy}rifamycin S
36-{[8-ethyl-5,8-dihydro-2-(4-methyl-1-piperazinyl)-5-oxopyrido[2,3-d]pyrimidin-6-yl]carbonyloxy} rifamycin S
36-{[1-cyclopropyl-6-fluoro-1,4-dihydro-7-(2,6-dimethyl-4-pyridiayl)-4-oxo-3-quinolinyl]carbonyloxy} rifamycin S
36-{[1-ethyl-1,4-dihydro-6-(4-methyl-1-piperazinyl)-4-oxo-3-pyridinyl]carbonyloxy} rifamycin S
36-[(1-ethyl-6-fluoro-1,4-dihydro-7-(4-methyl-1-piperazinyl)-4-oxo-3-quinolinyl)carbonyloxy]-3-(4-morpholinyl) rifamycin S
36-{[1-ethyl-1,4-dihydro-7-methyl-4-oxo-1,8-naphthyridin-3-yl]carbonyloxy]-3-(4-morpholinyl) rifamycin S
36-{[8-ethyl-5,8-dihydro-2-(4-methyl-1-piperazinyl)-5-oxopyrido[2,3-d]pyrimidin-6-yl]carbonyloxy}-3-(4-morpholinyl) rifamycin S
36-{[1-cyclopropyl-6-fluoro-1,4-dihydro-7-(2,6-dimethyl-4-pyridinyl)-4-oxo-3-quinolinyl]carbonyloxy}-3-(4-morpholinyl) rifamycin S
36-{[1-ethyl-1,4-dihydro-6-(4-methyl-1-piperazinyl)-4-oxo-3-pyridinyl]carbonyloxy}-3-(4-morpholinyl) rifamycin S
36-[(1-ethyl-6-fluoro-1,4-dihydro-7-(4-methyl-1-piperazinyl)-4-oxo-3-quinolinyl)carbonyloxy]-3-{[(4-methyl-1-piperazinyl)imino]methyl} rifamycin SV
36-{[1-ethyl-1,4-dihydro-7-methyl-4-oxo-1,8-naphthyridin-3-yl] carbonyloxy}-3-{[(4-methyl-1-piperazinyl)imino]methyl} rifamycin SV
36-{[8-ethyl-5,8-dihydro-2-(4-methyl-1-piperazinyl)-5-oxopyrido[2,3-d]pyrimidin-6-yl]carbonyloxy)-3-{[(4-methyl-1-piperazinyl)imino)methyl) rifamycin SV
36-{[1-cyclopropyl-6-fluoro-1,4-dihydro-7-(2,6-dimethyl-4-pyridinyl)-4-oxo-3-quinolinyl]carbonyloxy}-3-{[(4-methyl-1-piperazinyl)imino]methyl} rifamycin SV
36-{[1-ethyl-1,4-dihydro-6-(4-methyl-1-piperazinyl)-4-oxo-3-pyridinyl]carbonyloxy}-3-{[(4-methyl-1-piperazinyl)imino]methyl} rifamycin SV
36-[(1-ethyl-6-fluoro-1,4-dihydro-7-(4-methyl-1-piperazinyl)-4-oxo-3-quinolinyl)carbonyloxy]-3-{[(4-cyclopentyl-1-piperazinyl)imino]methyl} rifamycin SV
36-{[1-ethyl-1,4-dihydro-7-methyl-4-oxo-1,8-naphthyridin-3-yl] carbonyloxy}-3-{[(4-cyclopentyl-1-piperazinyl)imino]methyl} rifamycin SV
36-{[8-ethyl-5,8-dihydro-2-(4-methyl-1-piperazinyl)-5-oxopyrido[2,3-d]pyrimidin-6-yl]carbonyloxy}-3-{[(4-cyclopentyl-1-piperazinyl)imino]methyl} rifamycin SV
36-{[1-cyclopropyl-6-fluoro-1,4-dihydro-7-(2,6-dimethyl-4-pyridinyl)-4-oxo-3-quinolinyl]carbonyloxy}-3-{[(4-cyclopentyl-1-piperazinyl)imino]methyl} rifamycin SV
36-{[1-ethyl-1,4-dihydro-6-(4-methyl-1-piperazinyl)-4-oxo-3-pyridinyl]carbonyloxy}-3-{[(4-cyclopentyl-1-piperazinyl)imino)methyl} rifamycin SV
36-{[1-ethyl-1,4-dihydro-6-(dimethylamino)-4-oxo-3-pyridinyl]carbonyloxy}-3-{[(4-cyclopentyl-1-piperazinyl)imino]methyl) rifamycin SV
36-{[1-ethyl-1,4-dihydro-6-(dimethylamino)-5-fluoro-4-oxo-3-pyridinyl]carbonyloxy}-3-{[(4-cyclopentyl-1-piperazinyl)imino]methyl} rifamycin SV
36-{[1-ethyl-1,4-dihydro-6-(4-methyl-1-piperazinyl)-5-fluoro-4-oxo-3-pyridinyl]carbonyloxy}-3-{[(4-cyclopentyl-1-piperazinyl)imino]methyl} rifamycin SV
36-[(1-ethyl-6-fluoro-1,4-dihydro-7-(4-methyl-1-piperazinyl)-4-oxo-3-quinolinyl)carbonyloxy]-2'-(diethylamino) rifamycin P
36-{[1-ethyl-1,4-dihydro-7-methyl-4-oxo-1,8-naphthyridin-3-yl]carbonyloxy}-2'-(diethylamino) rifamycin P
36-{[8-ethyl-5,8-dihydro-2-(4-methyl-1-piperazinyl)-5-oxopyrido[2,3-d]pyrimidin-6-yl]carbonyloxy}-2'-(diethylamino) rifamycin P
36-{[1-cyclopropyl-6-fluoro-1,4-dihydro-7-(2,6-dimethyl-4-pyridinyl)-4-oxo-3-quinolinyl]carbonyloxy}-2'-(diethylamino) rifamycin P
36-{[1-ethyl-1,4-dihydro-6-(4-methyl-1-piperazinyl)-4-oxo-3-pyridinyl]carbonyloxy}-2'-(diethylamino) rifamycin P.

The preferred pharmaceutically acceptable salts of the compounds of formula I are the rifamycin salts formed with alkali metal or basic aminoacids; most preferred are the salts with sodium, arginine, lysine or histidine.

A further object of the present invention is to provide a process for preparing the compounds of general formula I.

All the suitable rifamycins known in the art may in general be used as starting materials for producing the compounds of the present invention.

Further chemical modifications of the above rifamycins, which allow to obtain the suitable starting material for producing the compounds of the invention, are known in the art, or can easily be carried out by the skilled man, according to the common general knowledge.

For instance, US 4086225 discloses the preparation of 4-desoxy-imidazolo[4,5-c] rifamycin derivatives from Rifamycin S; US 4880789 describes the preparation of 2'-dialkylamino derivatives of Rifamycin P.

Alternatively, 36-substituted rifamycin S or SV may be obtained according to the present process while the desired substituents are introduced in the other positions of the molecule only afterwards. This procedure is preferably followed when said further substituents would prevent the normal course of reaction or could itself undergo unwanted chemical modifications during the preparation of the 36-derivatives according to the process of the present invention.

For instance the presence of the group -CH=N-R¹⁰, wherein R¹⁰ is as defined in formula I, in position 3 would prevent the protection of positions 21 and 23 of the rifamycin molecule (i.e. the formation of the cyclic-21,23-(1-methylethylidene acetal)), which protection is necessary for obtaining the starting material of the present process. Therefore, after the desired 36-derivative of rifamycin SV has been obtained as described hereinafter, it will be further reacted, for instance with N-methylene-t-butylamine in the presence of t-butylamine and manganese dioxide and then with a compound of formula NH₂-R¹⁰, in order to insert at the 3- position the desired substituent of formula -CH=N-R¹⁰.

According to the common general knowledge, the skilled man will decide whether to introduce such further substituents before or after the process of the present invention, depending on the specific characteristics of the desired substituent.

The compounds of formula I may be obtained by reacting the corresponding 25-0-deacetylated rifamycin with a suitable malonic acid derivative in the presence of a condensing agent.

The deacetylation in position 25 of rifamycin, is easily achieved according to the common hydrolysis techniques known in the art; for instance, US 4188321, discloses the preparation of 25-O-Deacetylrifamycin S, or derivatives thereof, by reacting rifamycin S, or derivatives thereof, with sodium hydroxide or sodium bicarbonate, respectively.

For the process of the invention, the above rifamycin starting materials may not be reacted as such, but has to be protected on the positions 21 and 23, before reacting it. Such protection is performed according to the methods known in the art for protecting geminal hydroxy groups and results in a cyclization of the two oxygen atoms of the hydroxy functions in position 21 and 23. As a general procedure, it is preferred to perform the deacetylation in position 25 after the protection reaction.

For instance, 25-O-deacetylrifamycin S cyclic-21,23-(1-methylethylidene acetal)- wherein R¹ is oxo and R² is hydrogen - may be prepared by reacting rifamycin S with acetone and anhydrous cupric sulfate or with 2,2-dimethoxypropane and sulfuric acid, and then hydrolyzing with NaOH, according to "W. Kump and H. Birchel, Helv.Chim. Acta, 1973, 56, 2323"; the preparation of 25-O-deacetyl-3-(4-morpholinyl)rifamycin S cyclic-21,23-(1-methyl-ethylidene acetal) - wherein R¹ is oxo and R² is morpholinyl - by reacting 3-(4-morpholinyl) rifamycin S with 2,2-dimethoxypropane in anhydrous acetone and then hydrolyzing with NaOH, is described in "W. Wehrli et al., Journal of antibiotics, 1987, 40, 1733".

The protected rifamycin P may be prepared by reacting the 25-O-Deacetylrifamycin S cyclic-21,23-(1-methylethylidene acetal) with N-bromosuccinimide and 1,1-diethylthiourea in dimethylformamide, thus obtaining the corresponding 25-O-Deacetylrifamycin P cyclic-21,23-(1-methyl-ethylidene acetal).

The process of the present invention therefore comprises:
a) reacting a compound of general formula II 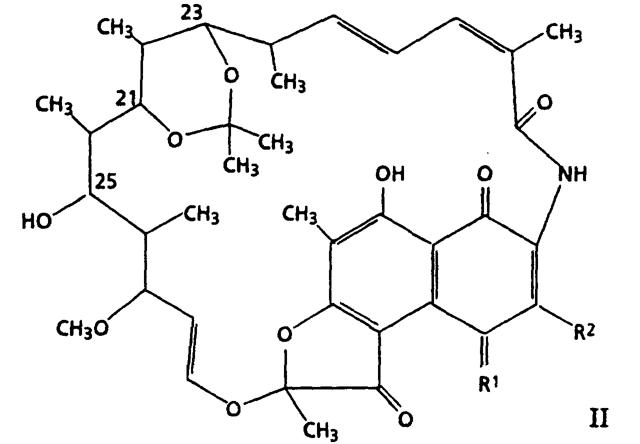 wherein R¹ and R² have the same meanings as in formula I, with the proviso that R² is not a group of formula:

   -CH=N-R¹⁰,

   with a malonic acid derivative of formula III: 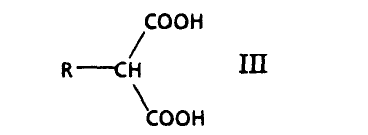 wherein R is as above defined, in the presence of a condensing agent;
b) removing the protecting group in positions 21 and 23 by means of an acidic cleavage of the acetonidic moiety;
c) contacting the deprotected compound with a cuprous salt or oxide or a mixture thereof in the presence of an inert organic solvent.

According to the first step of the above process one of the two malonic carboxylic moieties reacts with the hydroxy moiety in position 25 of the compound of formula II, thus forming an ester moiety in such position. The second carbon atom of this moiety is conventionally numbered as C³⁶.

This reaction is generally conducted in the presence of an inert organic solvent which do not unfavorably interfere with the reaction course and is capable of at least partially solubilizing the antibiotic material.

Said inert organic solvents are those commonly used in the art and comprise alkylamides, alkylnitriles, saturated linear or cyclic ethers, glycol ethers, phosphoramides, sulfoxides, chlorinated solvents or mixtures thereof.

Preferred inert organic solvents are:
dimethylformamide, acetonitrile, dimethoxyethane, tetrahydrofuran (THF), hexamethyl-phosphoramide, dimethylsulfoxide, chloroform and dichloroethane or mixtures thereof; most preferred is tetrahydrofuran.

The condensing agent may be any substance commonly used in the art for the esterification reactions selected from carboxydiimides, dialkylaminopyridines, carbonylimidazoles, triphenylphosphine in carbon tetrachloride, substituted dithiocarbonates and diphenylphosphorylazides. Examples of said condensating agents are: 4-dimethylaminopyridine, N,N'-carbonyl-bis imidazole, S,S'-bis-1-(phenyl-1H-tetrazol-5-yl)-dithiocarbonate and 1,3-Dicyclohexylcarbodiimmide (DCC).

Preferred condensing agents are carboxydiimmides derivatives, 1,3-Dicyclohexylcarbodiimmide being the most preferred one.

The above reaction is preferably conducted at temperatures from about 0°C to 35°C, while the reaction time may vary from 1 to 2 hours. More preferably, the mixture is reacted at 0°C for about 15 minutes and then for about one hour at room temperature.

The removal of the acetonidic moiety bridging positions 21 and 23 is performed under mild acidic conditions in the presence of an inert organic solvent, as above defined. The acids commonly used for the cleavage of such moiety can be here conveniently utilized; these acids have to be in diluted form, to avoid the demolition of the substrate. Suitable acids are mineral acids (e.g. hydrochloric acid, sulfuric acid) or organic sulfonic acids (e.g. p-toluensulfonic acid), the preferred one being sulfuric acid.

The reaction is conducted between 30°C and 50°C, while the reaction time may vary from 14 to 18 hours. It is generally preferred to carry it out at a temperature of about 40°C for about 16 hours.

Step c of the present process permits the removal of the free carboxy moiety linked to the C³⁶, for obtaining the corresponding compound of general formula 1.

For the decarboxylation reaction any salt or oxide or a mixture thereof containing the cuprous ion can be employed. Examples of these compounds are: Cu₂O, Cu₂S, CuCl, CuBr and Cu₂SO₄, most preferred being cuprous oxide.

Although all the cuprous compounds work well as decarboxylating agents, the use of CuCl or CuBr should be avoided when the malonic acid substrate is substituted with a bromine or chlorine atom respectively, because of a possible ion exchange side reaction. Of course, this problem arises only if the product has to be obtained in a pure form, while if a mixture of halogenated derivatives is desired, the use of CuCl or CuBr in this specific case is also possible.

The inert organic solvent to be used should be capable of at least partially solubilizing the antibiotic material and should not unfavorably interfere with the reaction course. The skilled man would be able to choose the most appropriate solvent among those commonly used in the art. Suitable solvents are those previously listed and, among those, the preferred solvent for this reaction is acetonitrile.

The reaction temperature is from 50°C to 75°C, preferably from 60°C to 70°C.

The reaction time may vary depending on the nature of substituent on the malonic moiety. For the most reactive compounds it is about one hour, while for the less reactive ones it raises up to 20 hours.

Although with the above described process it is possible to directly obtain the new rifamycin derivatives of the invention, some of these compounds are preferably obtained according to an alternative procedure, which comprises contacting a 36-halo rifamycin derivative obtained according to the above process with an appropriate reactant, in order to insert the desired substituent R in the position 36.

With this alternative procedure it is therefore not necessary to prepare the specific substituted malonic acid derivative of formula III according to each meaning of R, but starting from halo-malonic acid most of the compounds of the invention are easily obtainable.

This procedure is particularly suitable when the desired substituent R in the position 36 of rifamycin is hydroxy, iodo, (C₁-C₄)acyloxy, (C₁-C₄)alkylamino, di(C₁-C₄)alkylamino or a group of formula:

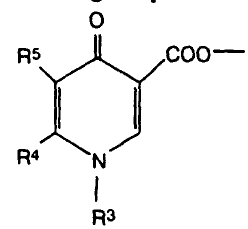

Such further reactions for preparing these new rifamycin derivatives are all subsequent to the previous steps a to c described above and may be either alternative or subsequent one to each other; these further steps are illustrated hereinafter for each kind of substituent.

When R is iodo, the 36-chloro or 36-bromo rifamycin derivative obtained according to steps a to c of the above process, is contacted with an acetonic solution of an alkali metal iodide.

The halogen ion exchange reaction for obtaining the 36-iodo rifamycin derivatives is easily accomplished by contacting the corresponding 36-halo rifamycin derivative with a metal alkali iodide, preferably sodium iodide, in the presence of acetone, as commonly known in the art.

Such reaction is generally conducted at room temperature for about 3 to 5 hours.

When R is (C₁-C₄)acyloxy, the 36-chloro, -bromo or -iodo rifamycin derivative obtained as above described, is contacted with a (C₁-C₄)acylate salt, in the presence of an inert organic solvent.

The (C₁-C₄)acylate salt is preferably an alkali metal salt, most preferred is potassium, or a silver salt, while the inert organic solvent is selected among those listed above, preferably being anhydrous dimethylformamide.

The reaction is generally conducted at room temperature for about 16 to 26 hours.

When R is (C₁-C₄)alkylamino or (C₁-C₄)dialkylamino the 36-chloro, -bromo or -iodo rifamycin derivative obtained as above described, is contacted with the correspondent alkyl or dialkylamine in the presence of an inert organic solvent.

The inert organic solvent is preferably tetrahydrofuran and the reaction is generally conducted at room temperature for about 2 to 6 hours.

When R is hydroxy, the 36-formyloxy rifamycin derivative obtained as above described, is hydrolyzed under basic conditions.

The hydrolysis is conducted in an hydroalcoholic solution under mild basic conditions, at room temperature, for about 10 to 16 hours. Preferably, the reaction is performed with potassium bicarbonate in a water/methanol mixture (ratio from 1ö2 to 1ö3 v/v).

Finally, when R is a group of formula 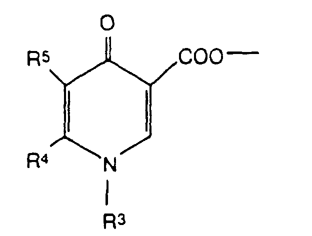 the 36-chloro, -bromo or -iodo rifamycin derivative obtained as above described is contacted with a salt of a 4-oxo-3-pyridinyl carboxylic acid derivative of general formula IV 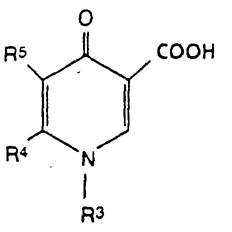 wherein R³, R⁴ and R⁵ are as defined in formula I, in the presence of an inert organic solvent.

Preferably a lithium, sodium, potassium or silver salt of the 4-oxo-3-pyridinyl carboxylic acid derivative is employed.

The 4-oxo-3-pyridinyl carboxylic acid derivatives, may be commercially available compounds such as Pefloxacin® , Nalidixic acid or N-methyl-pipemidic acid, known compounds such as those described in US 5075319, or new compounds derived from the compounds of general formula Va or Vb: 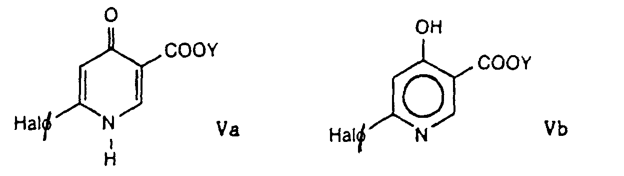 wherein Hal represents chloro or bromo and Y is hydrogen or lower alkyl. The compounds of formula IVa and IVb may be prepared by saponification of the corresponding 4,6-di-halo-nicotinic acid or lower alkyl 4,6-di-halo-nicotinate, as disclosed in Israeli Patent 44327/2.

The compounds of formula Va are preferably reacted first with an alkylating agent, so to obtain the corresponding N-(C₁-C₄)alkyl or N-(C₃-C₆)cycloalkyl derivatives; afterwards this N-alkylated compound is reacted with a suitable amine in order to substitute the halogen with the desired moiety of formula -NR⁶R⁷, wherein R⁶ and R⁷ are as defined in formula I.

For instance, suitable starting materials for the process of the present invention may be prepared by reacting a N-alkyl derivative of a compound of general formula IVa with N-methylpiperazine.

The novel compounds of general formula VI 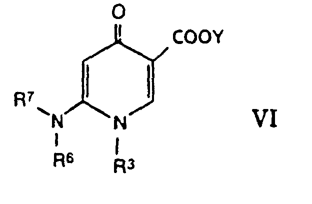 wherein Y is hydrogen or (C₁-C₄)alkyl and R³, R⁶ and R⁷ have the same meanings as in formula I, also fall within the scope of the present invention.

The inert organic solvent that may be used in the present process should be capable of at least partially solubilizing the antibiotic material and should not unfavourably interfere with the reaction course. The skilled man would be able to choose the most appropriate solvent among those commonly used in the art, partcularly in view of the teachings of the present disclosure. Suitable solvents are those listed before when dealing with reaction of the malonic acid derivative with rifamycin. Particularly preferred is dimethylformamide.

The reaction is conducted generally at a temperature between 15°C and 40°C.

The reaction time may vary from 10 to 24 hours.

Preferably, the salt of the compond of formula IV is first added to the inert organic solvent and stirred at room temperature for about 30 minutes, optionally in the presence of an activated molecular sieve, such as Union Carbide type 4Å (FLUKA); afterwards, the suitable 36-chloro, -bromo or -iodo rifamycin derivative is added to the solution and the mixture is stirred for from 14 to 20 hours at room temperature.

Preferred 36-halo rifamycin derivatives which may be employed for the present process are the 36-iodo derivatives, whilst preferred carboxylic acid salt is potassium salt.

Separation and purification of the reaction products obtained according to the various steps of the present process is made according to the known per se techniques.

The separation of the reaction products is preferably accomplished by means of extraction with water-immiscible organic solvents or by adding non-solvents.

The term "water-immiscible solvent" as used in this application, is intended to have the meaning currently given in the art to this term and refers to solvents that at the conditions of use are slightly miscible or practically immiscible with water in a reasonably wide concentration range, suitable for the intended use.

Examples of water-immiscible organic solvents that can be used in the extraction of the antibiotic substances of the invention from an aqueous phase are: the usual hydrocarbon solvents which may be linear, branched or cyclic such as hexane or cyclohexane; halogenated hydrocarbons such as chloroform, carbon tetrachloride, dichloromethane, dichloroethane, fluorobromoethane, dibromoethane, trichloropropane, chlorotrifluorooctane and the like; aromatic hydrocarbons such as benzene, toluene, xylene and the like; esters of at least four carbon atoms, such as ethyl acetate, propyl acetate, ethyl butyrate, and the like; alkanols of at least four carbon atoms which may be linear, branched or cyclic such as butanol, 1-pentanol, 2-pentanol, 3-pentanol, 1-hexanol, 2-hexanol, 3-hexanol, 3,3-dimethyl-1-butanol, 4-methyl-1-pentanol; 3-methyl-1-pentanol, 2,2-dimethyl-3-pentanol, 2,4-dimethyl-3-pentanol, 4,4-dimethyl-2-pentanol, 5-methyl-2-hexanol, 1-heptanol, 2-heptanol, 5-methyl-1-hexanol, 2-ethyl-1-hexanol, 2-methyl-3-hexanol, 1-octanol, 2-octanol, cyclopentanol, 2-cyclopentylethanol, 3-cyclopentyl-1-propanol, cyclohexanol, cycloheptanol, cyclooctanol, 2,3-dimethylcyclohexanol, 4-ethylcyclohexanol, cyclooctylmethanol, 6-methyl-5-hepten-2-ol, 1-nonanol, 2-nonanol, 1-decanol, 2-decanol and 3-decanol; straight or branched alkyl ethers and mixture thereof such as petroleum ether, ethyl ether, propyl ether, butyl ether, etc; and mixtures or functional derivatives thereof; the preferred one being ethyl acetate.

Examples of precipitating agents are petroleum ether and lower alkyl ethers, such as ethyl ether, propyl ether and butyl ether; the preferred one being petroleum ether.

Purification of the reaction products may be obtained by precipitation with non-solvents or by chromatographic techniques.

Precipitating agents suitable for purification are those listed above.

The chromatographic techniques suitable for purifying the reaction products of the present process are those commonly known in the art and comprise partition chromatography, reverse-phase partition chromatography, ion-exchange chromatography, flash chromatography, affinity chromatography, HPLC techniques and the like, the preferred one being flash chromatography. Preferably the purification of the residue is accomplished by means of flash-chromatography; particulary preferred as stationary phase is silica gel, while preferred eluent is methanol in dichloroethane.

The antimicrobial activity of the compounds of the present invention was demonstrated by a series of standard *in vitro* tests.

The minimal inhibitory concentration (MIC) for the microorganisms was determined by broth microdilution methodology. Inocula were approximately 10⁴ colony-forming units per ml (CFU/ml), except for Bacteroides fragilis, Clostridium perfringens and Propionibacterium acnes (10⁵ CFU/ml).

Incubation was at 37°C. Neisseria gonorrhoeae and Haemophilus influenzae were incubated in 5% carbon dioxide in air; C. perfringens, P. acnes and B. fragilis in nitrogen-carbon dioxide-hydrogen (80:10:10); other organisms in air. Incubation times were 48 hours for N. gonorrhoeae, H. influenzae, P. acnes and B. fragilis; 20-24 hours for other organisms.

The growth media were: Iso-Sensitest broth (Oxoid) for staphylococci, Enterococcus faecalis, Escherichia coli, Proteus vulgaris, Klebsiella pneumoniae, and Pseudomonas aeruginosa; Todd Hewitt broth (Difco) for streptococci; GC Base broth (Difco) + 1% (v/v) IsoVitaleX (BBL) for N. gonorrhoeae; Brain Heart Infusion broth (Difco) + 1% (v/v) Supplement C (Difco) for H. influenzae; Wilkins-Chalgren broth (Difco) for C. perfringens, P. acnes and B. fragilis.

The following table I reports the antimicrobial activity of representative compounds of the invention.

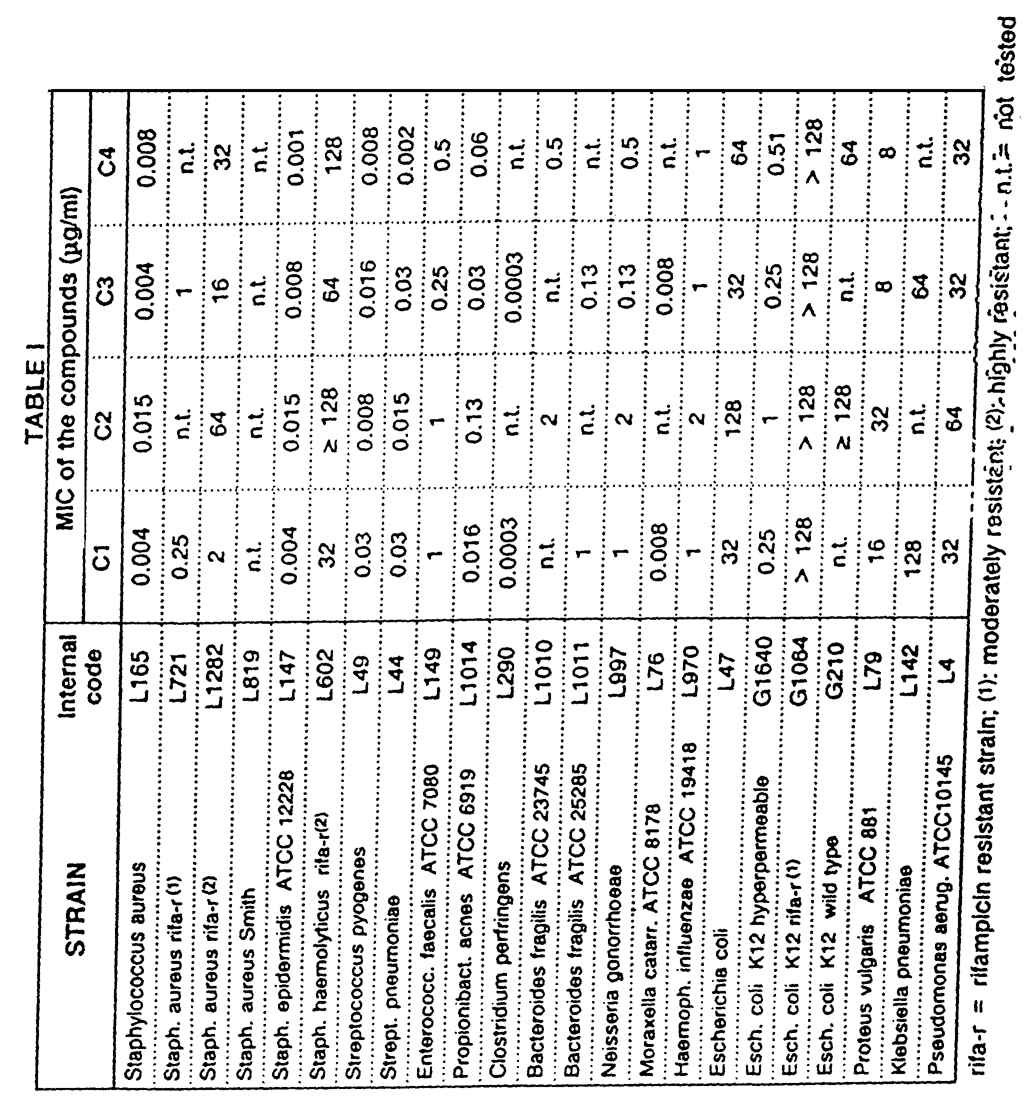

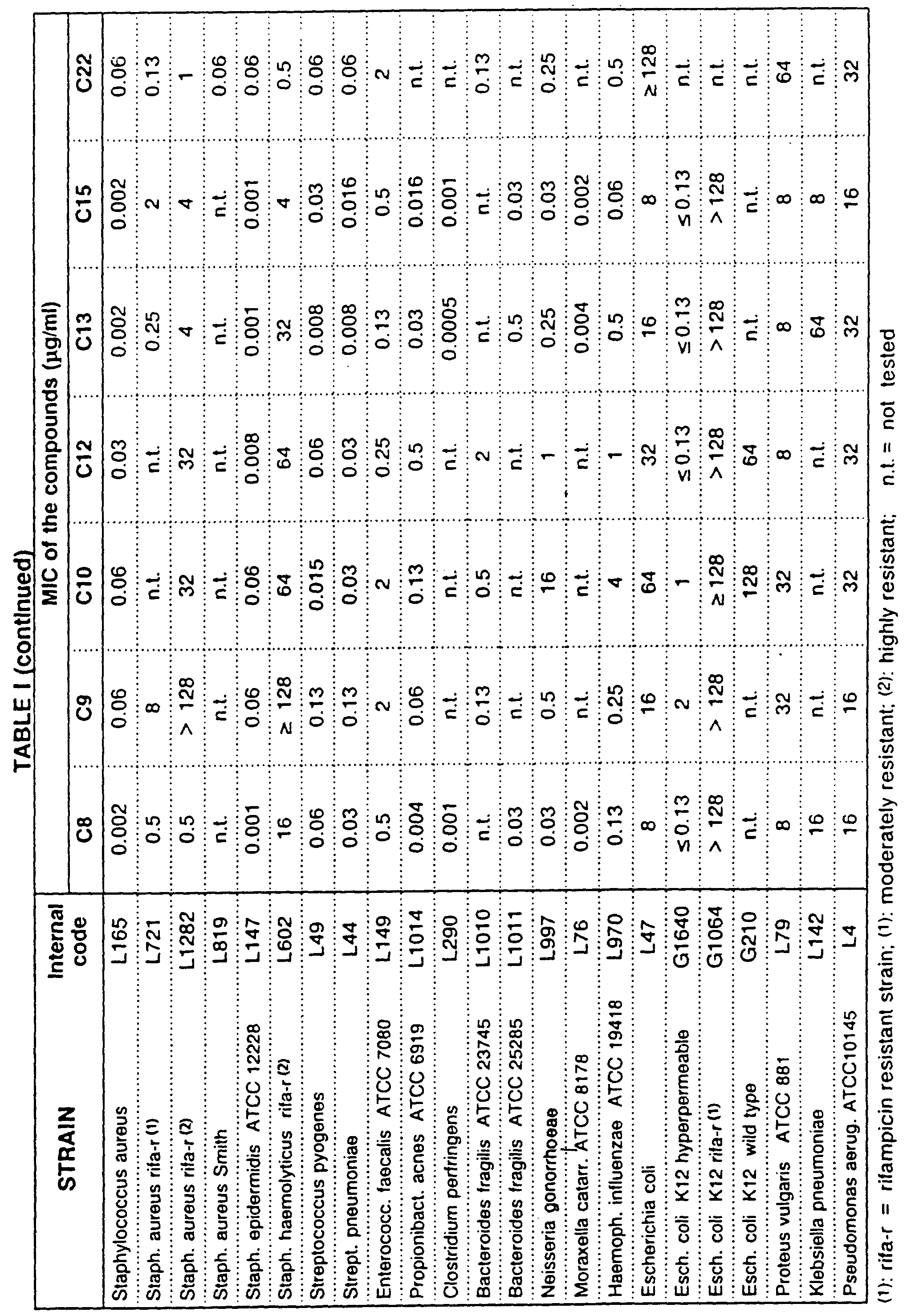

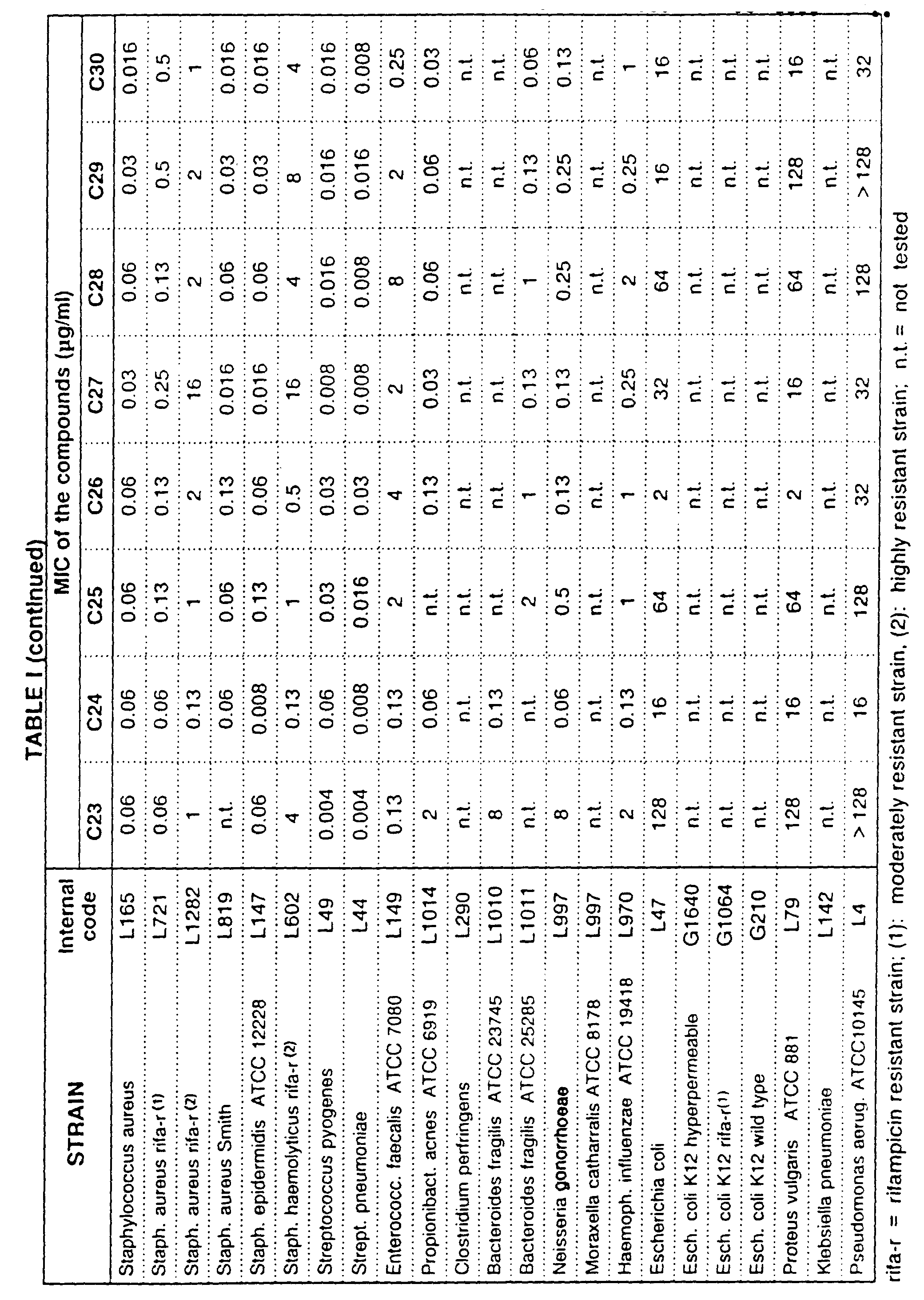

Compounds C22, C24, C25 and C30 have also been tested on various clinical isolates of rifampicin resistant strains of S. aureus, showing in the most of cases a MIC of from 0.125 to 2 µg/ml.

Compound C30 has further been tested against Mycobacterium tuberculosis and Mycobacterium avium, according to the following methodology:
M. tuberculosis was grown for 2-3 weeks on Lowenstein-Jensen medium (Sclavo) and M. avium on 7H10 agar (Difco) for 2 weeks. The cultures were suspended in 7H9 broth (Difco), diluted in Becton Dickinson diluting fluid, and inoculated into Bactec 12B vials (Becton Dickinson).

The inocula for control vials (without the compound to be tested) were 10²-10³ CFU/ml; vials with compound C30 were inoculated with 100 times this number of cells. The vials were incubated at 37°C and read daily in a Bactec 460 machine.

M. tuberculosis was considered sensitive to a given concentration of antibiotic if the difference between growth indexes on successive days in the vial was less than that of the control. M. avium was considered sensitive to a given antibiotic concentration if the growth index was less than that of the control.

The inhibitory concentration of compound C30 was found to be less than 0.2 against both the strains.

Some compounds of the invention were also tested in experimental septicemia in mice infected with Staphylococcus aureus Smith (Int. code L 819).

For this purpose, groups of five mice were infected intraperitoneally with about 1 x 10⁶ CFU of S. aureus Smith suspended in 0.5 ml of Difco bacteriological mucin.

Untreated animals died within 48 hours.

The other animals were treated once immediately after infection. On the 7th day the value of ED₅₀ (expressed in mg/kg) was calculated by the method of Spearman-Kärber (Finney, D.J., Statistical method in biological assay, page 254, C. Griffin Ltd., London, 1952), on the basis of the percentage of animals surviving at each dose.

The results are reported in the following table II:

**TABLEII**

| Experimental septicemia in mice infected with S. aureus Smith ( L 819) | | |
|---|---|---|
| **Compound** | **ED**_{**50**} **(mg/kg)** | |
| | **SC route** | **IV route** |
| C22 | 5.3 | 0.7 |
| C23 | 32.9 | n.t. |
| C24 | >50 | 3.8 |
| C25 | 4.7 | 2.9 |
| C26 | 1.6 | 0.95 |
| C30 | 1.6 | 0.72 |
| SC = Subcutaneous; IV= Intravenous; n.t. = not tested | | |

As shown in table II, when the compounds of the invention are administered by subcutaneous or intravenous routes, a good activity is observed in general. The activities of the above compounds were however negligible when administered orally.

In view of their properties, the compounds of the invention can be used as active ingredients in the preparation of medicaments for human or animal treatment.

In particular, the antibiotic compounds of general formula I are antimicrobial agents mainly active against gram positive bacteria, and fastidious gram negative bacteria.

Furthermore, the compounds of the invention show a considerable antimicrobial activity against those strain which have developed resistance to rifampicin.

The main therapeutic indication of the antibiotic substances of the invention is thus in the treatment of infections related to the presence of a microorganism susceptible to them.

The term "treatment" is intended to encompass also prophylaxis, therapy and cure.

The patient receiving this treatment is any animal in need, including primates, in particular humans, and other mammals such as equines, cattle, swine and sheep; and poultry and pets in general.

The compounds of the invention can be administered as such or in admixture with pharmaceutically acceptable carriers and can also be administered in conjunction with other antimicrobial agents generally employed in the art along with rifamycin antibiotics. Conjunctive therapy, thus includes sequential, simultaneous or separate administration of the active compound in a way that the therapeutical effects of the first administered one has not entirely disappeared when the subsequent one is administered.

The amount of active substance in the finished dosage form is related to a certain extent to the minimal inhibitory concentration of active substance against the infection causative agents and its particular type of formulation.

The dosage may obviously be adjusted according to the severity of the infection, the type, age and conditions of the patient, the formulation selected for the administration, the administration schedule, etc.

Experimental tests for determining the sensitivity of the microorganisms isolated from the patient may also offer useful indication to select the appropriate dosage.

In general, effective antimicrobial dosages are employed per single unit dosage form.

Repeated administrations, e.g. from 2 to 6 times a day, are in general preferred. An effective dosage may be in general in the range 0.5-100 mg/kg body weight/day, preferably 5-50 mg/kg body weight/day.

Anyway, the prescribing physician will be able to determine the optimal dosage for a given patient in a given situation.

The antibiotic compounds of the invention may be administered by parenteral (intramuscular, intravenous, subcutaneous, etc.) or oral route; the nature of the compound will determine the specific route of administration that may be employed. When the compound is not inactivated and is absorbed from the gastrointestinal tract, the oral route is generally preferred; otherwise, and also in the case of the unconscious or uncooperative state of the patient, parenteral administration of the active substance may conveniently be employed.

For oral administration the compounds of the invention can be formulated into solid or liquid preparations such as capsules, pills, tablets, troches, powder, solutions, suspensions or emulsions.

For instance, the solid unit dosage form can be a capsule of the ordinary gelatin type containing lubricants and inert filler, such as lactose, sucrose and cornstarch. In another embodiment, the compounds of general formula I can be tableted with conventional tablet bases such as lactose, sucrose and cornstarch in combination with binders, such as acacia, cornstarch or gelatin, disintegrating agents such as potato starch or alginic acid and a lubricant such as stearic acid or magnesium stearate.

A unit dosage for oral administration may contain, for instance, from 50 to 700 mg of the active ingredient, preferably about 150 to 500 mg of the active ingredient.

For parenteral administration the compounds of the invention may be formulated into suitable injectable preparations containing a liquid vehicle. Such vehicle normally has no therapeutic effect and should not be toxic. Examples of suitable vehicles for preparing injectable dosage forms of the compounds of the invention are water, aqueous vehicles (e.g. Sodium chloride injections, Dextrose injections, etc.), water miscible solvents (e.g. ethyl alcohol, polyethylene glycol, propylene glycol, etc.) and non-aqueous vehicles (e.g. "fixed oils" such as corn oil, cottonseed oil, peanut oil and sesame oil). Optionally, the injectable preparation may further contain buffers for stabilizing the solution (e.g. citrates, acetates and phosphates) and/or antioxidants (e.g. ascorbic acid or sodium bisulfite).

Also in the case of parenteral administration of the compounds of the invention, the nature of the product will determine the specific route of administration (e.g. intramuscular, intravenous or subcutaneous) that may be employed. Conversely, the desired route of administration will place requirements on the formulation. For example, suspensions would not be administered directly in the blood stream because of the danger of insoluble particles blocking capillaries, whilst solutions to be administered subcutaneously would require strict attention to tonicity adjustment, otherwise irritation of the nerve endings in the anatomical area would give rise to pronounced pain.

Useful indications for the preparations of suitable parenteral and oral dosage forms can be found in: Remington's Pharmaceutical Sciences, 17th Edition, 1985 (Hack Publishing Company, Easton, Pennsylvania).

Besides their use as medicaments in human and veterinary therapy, the compounds of the invention may also be used as animal growth promoters.

For this purpose, a compound of the invention is administered orally in a suitable feed. The exact concentration employed will reflect the amount of active agent needed for a growth promotant effect and the amount of feed normally consumed.

The addition of the active compound of the invention to animal feed is preferably accomplished by preparing an appropriate feed premix containing the active compound in an efficacious amount and incorporating the premix into the complete ration. Alternatively, an intermediate concentrate or feed supplement containing the active ingredient can be blended into the feed.

The way in which such feed premixes and complete rations can be prepared and administered, is described in reference books (such as "Applied Animal Nutrition", W.H. Freedman and CO., S. Francisco, USA, 1969 or "Livestock Feeds and Feeding", O and B books, Corvallis, Oregon, USA, 1977).

To better illustrate the present invention, examples of synthesis of compounds of general formula I are given hereinafter.

For the sake of clarity, the preparation of the starting material, of the intermediates and of the compounds of formula I has been so divided:
- Preparations A to G refer to the preparation of the starting materials (the compounds obtained are marked with letter P, i.e. P1 to P7);
- Examples A to I refer to the preparation of intermediate compounds (the compounds obtained are marked with letter E, i.e. E1 to E9);
- Examples 1 to 30 refer to the preparation of the compounds of formula I, (the compounds obtained are marked with letter C, i.e. C1 to C30).

In the following examples, the reaction is followed by means of TLC plates (silica-gel 60 F₂₅₄ precoated, 5x10 cm, thickness 0.25, Merck); the purification of the products obtained by flash-chromatography is performed on silica gel (32-63, 60 Å; ICN Biomedicals GmbH), with methanol in dichloromethane as the eluent.

### Preparation A: 25-O-Desacetyl-3-(4-morpholinyl)rifamycin S cyclic-21,23-(1-methylethylidene acetal) (Compound P1)

a) A mixture of rifamycin S (15 g), morpholine (40 ml) and dioxane (40 ml) is stirred 2 hours at room temperature. The mixture is cooled in icy water and 5N hydrochloric acid (45 ml) is added. Extraction with ethyl acetate (2 x 100 ml) and evaporation of the solvent give an oily residue which is dissolved in chloroform (100 ml) and stirred 2 hours with a 25% solution of potassium hexacyanoferrate (III) in water (100 ml). The organic phase is separated, washed with water, dried and evaporated to dryness. The residue is purified by flash-chromatography; elution with 2% methanol in dichloromethane gives pure 3-(4-morpholinyl) rifamycin S, of which, after crystallization from ethyl ether/ petroleum ether, 13.8 g are obtained;
m.p. 180-185°C (dec).
TLC (methanol:dichloromethane, 5:95): black spot,
Rf 0.39.
b) Two drops of concentrated sulfuric acid are added to a mixture of 3-(4-morpholinyl) rifamycin S (11 g), 2,2-dimethoxypropane (11 ml) and dry acetone (120 ml). The reaction mixture is stirred 45 minutes at room temperature. Anhydrous sodium carbonate (1 g) is added and stirring continued for 5 min. The solution is filtered and evaporated to dryness. The residue is purified by flash-chromatography; elution with 1% methanol in dichloromethane affords 7.3 g of pure 3-(4-morpholinyl)rifamycin S cyclic-21,23-(1-methylethylidene acetal) which is dissolved in a cold solution of 5% NaOH in methanol (100 ml). The resulting mixture is stirred 18 hours at room temperature then diluted with icy water (100 ml), acidified (about pH 4) with citric acid and extracted with dichloromethane (3 x 100 ml). The combined extracts are dried and evaporated to dryness. The residue, by crystallization from ethyl ether/petroleum ether, gives the pure title compound (6.4 g) m.p. 171-174°C (dec).
TLC (methanol:dichloromethane, 5:95): black spot,
Rf 0.33.

| Analysis for C₄₂H₅₄N₂O₁₂, MW = 778.904 | | | |
|---|---|---|---|
| Calculated | C 64.76 | H 6.99 | N 3.59 |
| Found | C 64.57 | H 7.01 | N 3.39 |

### Preparation B: 25-O-Desacetylrifamycin S cyclic-21,23-(1-methylethylidene acetal) (Compound P2)

The title compound is prepared as described by W. Kump and H. Birchel; Helv. Chim. Acta, 1973, 56, 2323.

### Preparation C: 25-O-Deacetyl-2'-(diethylamino)rifamycin P cyclic-21,23-(1-methylethylidene acetal) (Compound P3)

N-Bromosuccinimide (2.2 g) in dimethylformamide (5 ml) is slowly dropped into a cold (5°C) solution of Compound P2 (7.7 g) and triethylamine (1.25 g) in dimethylformamide (25 ml). The reaction mixture is stirred for 3 hours at room temperature, then 1,1-diethylthiourea (1.8 g) in dimethylformamide (4 ml) is added. Stirring is continued for 1.5 h, then ascorbic acid (2.6 g) in water (5 ml) is added. The mixture is allowed to rest overnight and then is poured into water (300 ml) and extracted with ethyl acetate (3 x 100 ml). The organic extracts are combined, washed with brine (200 ml), dried and evaporated to dryness. The residue is purified by flash-chromatography; elution with 1.8% methanol in dichloromethane gives the pure title compound (6.5 g).
TLC (methanol:dichloromethane, 1:9): orange spot,
Rf 0.54.

| Analysis for C₄₃H₅₇N₃O₁₀S, MW = 808.031 | | | |
|---|---|---|---|
| Calculated | C 63.92 | H 7.11 | N 5.20 |
| Found | C 63.59 | H 7.26 | N 5.06 |

### Preparation D: 1-Ethyl-1,4-dihydro-6-(4-methyl-1-piperazinyl)-4-oxo-3-pyridinecarboxylic acid dihydrochloride (Compound P4)

a) ethyl 4,6-dichloronicotinate (17 g) is boiled for 4 hours with 24% sulfuric acid (400 ml). The white crystals that separate are filtered from the boiling solution and 4-chloro-6-hydroxynicotinic acid (6 g, m.p. 299-300°C) is obtained; the acidic solution is left overnight in the refrigerator. The crystallized solid is collected by filtration and dried under vacuum, thus obtaining 6-chloro-4-hydroxynicotinic acid (4.6 g) m.p. 231-233°C
b) A mixture of 6-chloro-4-hydroxynicotinic acid (4 g), absolute ethanol (120 ml), toluene (60 ml) and sulfuric acid (13 ml) is gently refluxed for 8 hours. After cooling the solvent is distilled off and the residue treated with icy water (100 ml). The solution is neutralized with a concentrated solution of Na₂CO₃ and then extracted with dichloromethane (3 x 100 ml). The combined extracts are dried over sodium sulfate and evaporated to dryness. The residue is dissolved in boiling ethyl ether (10 ml) then petroleum ether is added (10 ml); on cooling, ethyl 6-chloro-4-hydroxy-nicotinate (3.8 g) mp 59-61°C.
c) A mixture of ethyl 6-chloro-4-hydroxynicotinate (3.7 g), powdered potassium carbonate (3.7 g), iodoethane (5 ml) and dimethylformamide (50 ml) is well stirred for 7 hours at 90°C. After cooling, the reaction mixture is filtered and the solvent evaporated off. The residue is treated with icy water (30 ml) and extracted with dichloromethane (2 x 30 ml). The combined extract are dried and evaporated to dryness. The residue is boiled 90 minutes with 1N sodium hydroxide (18 ml). After cooling the solution is extracted with diethyl ether (30 ml), that is discarded. The alkaline solution is concentrated to small volume, cooled and acidified to pH 4 with 1 N hydrochloric acid. The white material that precipitates is collected and crystallized from ethanol to give 6-chloro-1,4-dihydro-1-ethyl-4-oxo-3-pyridine-carboxylic acid (3 g) m.p. 155-57°C.
d) 6-chloro-1,4-dihydro-1-ethyl-4-oxo-3-pyridine-carboxylic acid (1.8 g) and N-methylpiperazine (5 ml) are stirred 5 hours at 130°C. The excess of amine is distilled off and the residue dissolved in 1M hydrochloric acid (10 ml). The acidic solution is evaporated to dryness and the residue crystallized from ethanol (4 ml). The alcoholic solution is kept overnight in the refrigerator, thus obtaining the title compound (1.1 g), m.p. 224-226°C.

| Analysis for C₁₃H₁₉N₃O₃.2HCl MW = 338.236 | | |
|---|---|---|
| Calculated | N 12.42 | Cl 20.96 |
| Found | N 11.97 | Cl 20.80 |

### Preparation E: 1-Cyclopropyl-7-(2,6-dimethyl-4-pyridinyl)-6-fluoro-1,4-dihydro-4-oxo-3-quinoline carboxylic acid (Compound P5)

a) Acetyl chloride (17 ml) is slowly dropped into a stirred mixture of 1-bromo-2,5-difluorobenzene (30 g) and aluminium trichloride (53 g) heated at 60°C and kept under argon. The reaction mixture is then stirred 90 minutes at 95°C. The mixture is cooled at 40°C and carefully poured into crushed ice (400 g) and concentrated hydrochloric acid (35 ml). The resulting mixture is stirred a few minutes and then extracted with ethyl ether (2 x 250 ml). The ethereal solution is washed to neutral with brine, dried over sodium sulfate and the solvent is evaporated. The distillation of the oily residue gives 4'-bromo-2',5'-difluoroacetophenone (21.2 g) b.p.(0,05 mm) = 60°C
b) Sodium hydride (7.1 g, 55% in mineral oil) is added in small portions to a well stirred solution of 4'-bromo-2',5'-difluoroacetophenone (20.8 g) in diethylcarbonate cooled at 5°C. The resulting mixture is stirred 10 minutes at 5°C and 90 minutes at 80°C. After cooling the reaction mixture is poured into crushed ice (800 g) and acetic acid (35 ml), then extracted with ethyl ether (3 x 200 ml). The combined extracts are washed to neutral with brine, dried over sodium sulfate and evaporated to dryness. The residue is purified by column chromatography containing 450 g of silica-gel (silica gel 60, particle size 0.063-0.200 mm Merck). Elution with 18% ethyl acetate in petroleum ether allows the separation of the less polar compound that, after crystallization from n-hexane gives ethyl 4-bromo-2,6-difluorobenzoylacetate (5.6 g) m.p. 49-52°C.
c) Dimethylformamide dimethylacetal (2.4 ml) is slowly dropped into a stirred solution of ethyl 4-bromo-2,6-difluorobenzoylacetate (5.4 g) in dry tetrahydrofuran (15 ml). Stirring is continued overnight at room temperature, then the solvent is evaporated. The reddish oily residue is dissolved in dry tetrahydrofuran (22 ml) and cooled to 0°C; cyclopropylamine (1.3 ml) is added and the resulting solution stirred 1 hour at 0°C. The solvent is evaporated under vacuum at room temperature. The residue, dissolved in dimethylformamide (25 ml), is stirred 1 hour at 100°C with anhydrous potassium carbonate (4.5 g). After cooling the solution is filtered and evaporated to dryness. The residue is triturated with water, collected and recrystallized from ethanol to give ethyl 7-bromo-1-cyclopropyl-6-fluoro-1,4-dihydro-4-oxo-3-quinolinecarboxylate (3.2 g), m.p. 251-253°C.
d) A mixture of 2,6-lutidine-N-oxide hydrochloride (48 g) and phosphorus oxychloride (110 ml) is heated 6.5 hours at reflux. After cooling the reaction mixture is carefully poured into crushed ice (1 kg). While the temperature is maintained below 15°C, concentrated ammonium hydroxide is added up to reach pH 8. The product that separates is extracted with ethyl ether (2 x 500 ml) which is dried and distilled off. The residue is dissolved in ethanol (400 ml) and boiled 3 hours with triethylamine (20 ml). After cooling, the solution is evaporated to dryness, treated with water (200 ml) and extracted with ethyl ether (4 x 150 ml). The combined extracts are dried and the solvent evaporated. The residue after distillation gives 4-chloro-2,6-dimethylpyridine (25 g), b.p.(15 mm) = 67-69°C. To a mixture of sodium (10 g, 30% dispersion in toluene) in anhydrous dimethyoxyethane (40 ml) cooled in an ice-bath and kept under argon, trimethyltin chloride (12.1 g) in dimethoxymethane (6 ml) is added over a one hour period while keeping the temperature at 0°C. The mixture is stirred 2.5 hours at 0°C; then 4-chloro-2,6-dimethylpyridine (7 g) in dimethoxyethane is slowly dropped into. Stirring is continued for an additional hour then the reaction mixture is allowed to stand overnight at room temperature. The reaction mixture is diluted with ethyl ether (100 ml) and filtered. The solvent is evaporated, the residue dissolved in ethyl ether (100 ml) and filtered again. After evaporation of the solvent the residue is distilled giving 2,6-dimethyl-4-(trimethyl-stannyl)pyridine (8.8 g), b.p.(15 mm) = 130-140°C.
e) 2,6-dimethyl-4-(trimethylstannyl)pyridine (2.6 g) in dioxane (6 ml) is slowly dropped into a stirred mixture of ethyl 7-bromo-1-cyclopropyl-6-fluoro-1,4-dihydro-4-oxo-3-quinolinecarboxylate (3.1 g), dioxane (55 ml) and hexamethylenphosphoramide (2.6 ml) kept under argon. To the stirred mixture, dichlorobis (triphenylphosphine) palladium (0.4 g) is added. The reaction mixture is heated under reflux for 24 hours. After cooling it is poured in water (200 ml) and repeatedly extracted with dichloromethane. The organic extracts are dried and evaporated to dryness. The residue, after trituration with ethyl ether, gives ethyl 1-cyclopropyl-7-(2,6-dimethyl-4-pyridinyl)-6-fluoro-1,4-dihydro-4-oxo-3-quinolinecarboxylate (2.4 g) m.p. 196-199°C.
f) A suspension of ethyl 1-cyclopropyl-7-(2,6-dimethyl-4-pyridinyl)-6-fluoro-1,4-dihydro-4-oxo-3-quinolinecarboxylate (2.3 g) in 1.5% NaOH (50 ml) is heated 2.5 h at reflux. The solution is decolorized with charcoal (1 g), filtered, cooled in an ice-bath and acidified with acetic acid. The separated solid is collected by filtration and crystallized from ethanol/chloroform to give the title compound (1.66 g) m.p. 300-303°C.

### Preparation F: Potassium salt of 1-ethyl-1,4-dihydro-6-(4-methyl-1-piperazinyl)-4-oxo-3-pyridinecarboxylic acid (Compound P6)

The potassium salt of compound P4 is prepared by adding 1N potassium hydroxide (6 ml) to the free acid dihydrochloride (676 mg - 2mmol) dissolved in water (5 ml). The aqueous solution is evaporated to dryness and the residue treated with boiling ethanol (20 ml). The insoluble material (KC1) is filtered and the alcoholic solution re-evaporated to dryness. The solid residue is triturated with diethyl ether, collected by filtration and dried over phosphorous pentoxide under vacuum giving the desired potassium salt (575 mg).

### Preparation G: Potassium salt of 1-Cyclopropyl-7-(2,6-dimethyl-4-pyridinyl)-6-fluoro-1,4-dihydro-4-oxo-3-quinoline carboxylic acid (Compound P7)

The potassium salt of compound P5 is prepared by adding 1N potassium hydroxide (2 ml) to the free acid (705 mg - 2mmol) dissolved in ethanol (10 ml). The resulting solution is evaporated to dryness and the solid residue is triturated with a 1:1 mixture of diethyl ether/ethanol, collected by filtration and dried over phosphorous pentoxide under vacuum giving the desired potassium salt (672 mg).

### Example A: 36-Bromo-36-carboxyrifamycin S (Compound E1)

a) 1,3-Dicyclohexylcarbodiimide (950 mg, 4.6 mmol) dissolved in dry tetrahydrofuran (2 ml) is slowly dropped into a stirred mixture of compound P2 (940 mg, 1.4 mmol), bromomalonic acid (840 mg, 4.6 mmol) and dry tetrahydrofuran (15 ml) cooled at 0°C. The reaction mixture is stirred 15 minutes at 0°C, then 1 hour at room temperature. The dicyclohexylurea that forms is filtered off and the solution evaporated to dryness. The oily residue is dissolved in ethyl acetate (20 ml) and washed with water (2 x 20 ml). After drying over sodium sulfate, the solvent is evaporated and the residue purified by flash-chromatography. Elution with 4% methanol in dichloromethane gives pure (36-Bromo-36-carboxy)rifamycin S cyclic-21,23-(1-methylethylidene acetal) (680 mg).
TLC (methanol:dichloromethane, 15:85): Rf 0.34
b) A mixture of the above obtained compound (680 mg), tetrahydrofuran (8 ml) and 3% sulfuric acid (3 ml) is stirred at 40°C for 16 hours. After cooling, the reaction mixture is poured into water (20 ml) and extracted with ethyl acetate (2 x 20 ml). The organic extracts are washed with brine to neutral, then dried and concentrated to a small volume. Dilution with petroleum ether gives a brownish precipitate which is collected and dried under vacuum yielding the title compound (490 mg).
TLC (methanol:dichloromethane, 15:85): Rf 0.27

| Analysis for C₃₈H₄₄BrNO₁₄, MW = 818.682 | | |
|---|---|---|
| Calculated | C 55.75 H 5.41 | N 1.85 Br 9.76 |
| Found | C 55.85 H 5.60 | N 1.70 Br 8.98 |

### Example B: 36-Carboxy-36-fluororifamycin S (Compound E2)

By following the procedure of Example A, but starting from Compound P2 (0.7 g) and fluoromalonic acid, the title compound is obtained (0.22 g).
TLC (methanol:dichloromethane, 15:85): Rf 0.20

| Analysis for C₃₈H₄₄FNO₁₄, MW = 757.771 | | | |
|---|---|---|---|
| Calculated | C 60.23 | H 5.85 | N 1.71 |
| Found | C 60.00 | H 5.94 | N 1.70 |

### Example C: 36-Carboxy-36-chlororifamycin S (Compound E3)

By following the procedure of Example A, but starting from P2 (9.6 g) and chloromalonic acid, the title compound is obtained (9.3 g).
TLC (methanol:dichloromethane, 2:8): Rf 0.45

| Analysis for C₃₈H₄₄ClNO₁₄, MW = 774.226 | | | | |
|---|---|---|---|---|
| Calculated | C 58.95 | H 5.73 | N 1.81 | Cl 4.58 |
| Found | C 58.49 | H 5.61 | N 1.73 | Cl 4.80 |

### Example D: 36-Carboxy-36-methylthiorifamycin S (Compound E4)

By following the procedure of Example A, but starting from Compound P2 (0.96 g) and methylthiomalonic acid, the title compound is obtained (0.47 g).
TLC (methanol:dichloromethane, 2:8): Rf 0.39

| Analysis for C₃₉H₄₇NO₁₄S, MW = 785.873 | | | | |
|---|---|---|---|---|
| Calculated | C 59.61 | H 6.03 | N 1.78 | S 4.08 |
| Found | C 59.14 | H 6.10 | N 1.76 | S 3.70 |

### Example E: 36-Carboxy-36-ethylrifamycin S (Compound E5)

By following the procedure of Example A, but starting from Compound P2 (0.96 g) and ethylmalonic acid, the title compound is obtained (0.40 g).
TLC (methanol:dichloromethane, 15:85): Rf 0.34

| Analysis for C₄₀H₄₉NO₁₄, MW = 767.836 | | | |
|---|---|---|---|
| Calculated | C 61.01 | H 6.43 | N 1.82 |
| Found | C 60.58 | H 6.50 | N 1.79 |

### Example F: 36-Carboxy-36-butylrifaaycin S (Compound E6)

By following the procedure of Example A, but starting from Compound P2 (0.96 g) and butylmalonic acid, the title compound is obtained (0.4 g).
TLC (methanol:dichloromethane, 1:9): Rf 0.51

| Analysis for C₄₂H₅₃NO₁₄, MW = 795.889 | | | |
|---|---|---|---|
| Calculated for | C 63.38 | H 6.71 | N 1.76 |
| Found | C 62.96 | H 6.75 | N 1.70 |

### Example G: 36-Carboxy-36-octylrifamycin S (Compound E7)

By following the procedure of Example A, but starting from Compound P2 (0.96 g) and octylmalonic acid, the title compound is obtained (0.66 g).
TLC (methanol:dichloromethane, 1:9): Rf 0.55

| Analysis for C₄₆H₆₁NO₁₄, MW = 851.998 | | | |
|---|---|---|---|
| Calculated | C 64.85 | H 7.22 | N 1.64 |
| Found | C 64.47 | H 7.30 | N 1.62 |

### Example H: 36-Bromo-36-carboxy-3-(4-morpholinyl)-rifamycin S (Compound E8).

By following the procedure of Example A, but starting from Compound P1 (32 g) and bromomalonic acid, the title compound is obtained (26 g).
TLC (methanol:dichloromethane, 2:8): Rf 0.43

| Analysis for C₄₂H₅₁BrN₂O₁₅, MW = 903.787 | | | | |
|---|---|---|---|---|
| Calculated | C 55.82 | H 5.69 | N 3.10 | Br 8.84 |
| Found | C 55.30 | H 5.70 | N 3.04 | Br 8.46 |

### Example I: 36-Bromo-36-carboxy-2'-(diethylamino)-rifamycin P (Compound E9)

By following the procedure described in Example A, from Compound P3 (3.3 g) and bromomalonic acid (2.5 g), the title compound (1.4 g) is obtained.
TLC (methanol:dichloromethane, 25:75): orange spot
Rf 0.35.

| Analysis for C₄₃H₅₄BrN₃O₁₃S, MW = 932.894 | | | | |
|---|---|---|---|---|
| Calculated | C 53.36 | H 5.83 | N 4.50 | Br 8.61 |
| Found | C 54.01 | H 5.93 | N 4.61 | Br 8.04 |

### Example 1: 36-Bromorifamycin S (Compound C1)

Compound E1 (3.6 g) dissolved in anhydrous acetonitrile (40 ml) is very slowly added to a well stirred suspension of cuprous (I) oxide (Cu₂O) (100 mg) in anhydrous acetonitrile (160 ml) heated at 60°C and kept under argon. The resulting mixture is stirred 1 hour at 60°-70°C. After cooling, it is filtered and evaporated to dryness. The residue, dissolved in ethyl acetate (40 ml), is washed with 1N hydrochloric acid (3x20 ml) and with brine to neutral. The organic phase is dried, the solvent evaporated off and the residue purified by flash-chromatography; elution with 1.2% methanol in dichloromethane allowed the isolation of the pure title compound (1.6 g).
TLC (methanol:dichloromethane, 1:9): Rf 0.61

| Analysis for C₃₇H₄₄BrNO₁₂, MW = 774.673 | | | | |
|---|---|---|---|---|
| Calculated | C 57.36 | H 5.73 | N 1.80 | Br 10.31 |
| Found | C 57.60 | H 6.10 | N 1.76 | Br 10.24. |

### Example 2: 36-Fluororifamycin S (Compound C2)

By following the procedure of Example 1, but starting from Compound E2 (0.14 g) and Cu₂O (0.01 g), the title compound is obtained (0.04 g).
TLC (methanol:dichloromethane, 1:9): Rf 0.65

| Analysis for C₃₇H₄₄FNO₁₂, MW = 713.763 | | | |
|---|---|---|---|
| Calculated | C 62.26 | H 6.21 | N 1.96 |
| Found | C 61.60 | H 6.34 | N 2.04 |

### Example 3: 36-Chlororifamycin S (Compound C3)

By following the procedure of Example 1, but starting from Compound E3 (18.6 g) and Cu₂O (0.48 g), the title compound is obtained (9.3 g).
TLC (methanol:dichloromethane, 1:9): Rf 0.68

| Analysis for C₃₇H₄₄ClNO₁₂, MW = 730.217 | | | | |
|---|---|---|---|---|
| Calculated | C 60.86 | H 6.07 | N 1.92 | Cl 4.85 |
| Found | C 61.00 | H 6.30 | N 1.76 | Cl 4.73 |

### Example 4: 36-Methylthiorifamycin S (Compound C4)

By following the procedure of Example 1, but starting from Compound E4 (0.43 g) and Cu₂O (0,03 g), the title compound is obtained (0.22 g).
TLC (methanol:dichloromethane, 1:9): Rf 0.54

| Analysis for C₃₈H₄₇NO₁₂S, MW = 741.863 | | | | |
|---|---|---|---|---|
| Calculated | C 61.52 | H 6.38 | N 1.88 | S 4.32 |
| Found | C 61.54 | H 6.75 | N 1.90 | S 4.30 |

### Example 5: 36-Bromo-3-(4-morpholinyl)rifamycin S (Compound C8)

By following the procedure of Example 1, but starting from Compound E8 (26 g) and Cu₂O (0.70 g), the title compound is obtained (11.8 g).
TLC (methanol:dichloromethane, 1:9): Rf 0.61

| Analysis for C₄₁H₅₁BrN₂O₁₃, MW = 859.777 | | | | |
|---|---|---|---|---|
| Calculated | C 57.27 | H 5.99 | N 3.26 | Br 9.29 |
| Found | C 56.91 | H 6.03 | N 3.07 | Br 8.81 |

### Example 6: 36-Bromo-2'-(diethylamino)rifamycin P (Compound C9)

By following the procedure described in Example 1, from Compound E9 (1.6 g) and Cu₂O (80 mg), the title compound (0.69 g) is obtained.
TLC (methanol:dichloromethane, 1:9): orange spot Rf 0.5.

| Analysis for C₄₂H₅₄BrN₃O₁₁S, HW = 888.884 | | | | |
|---|---|---|---|---|
| Calculated | C 56.75 | H 6.12 | N 4.72 | Br 8.99 |
| Found | C 55.89 | H 6.05 | N 4.68 | Br 9.30 |

### Example 7: 36-Iodorifamycin S (Compound C10)

Sodium iodide (NaI) (0.47 g) dissolved in acetone (3 ml) is added to Compound C1 (1.12 g) dissolved in acetone (8 ml). The resulting mixture is stirred at room temperature for 4 hours. The solvent is evaporated off and the residue purified by flash-chromatogarphy. Elution with 1.42 methanol in dichloromethane gives pure title compound (1.1 g).
TLC (methanol:dichloromethane, 1:9): Rf 0.59

| Analysis for C₃₇H₄₄INO₁₂, MW = 821.668 | | | |
|---|---|---|---|
| Calculated | C 54.08 | H 5.40 | N 1.70 |
| Found | C 54.01 | H 6.00 | N 1.85 |

### Example 8: 36-Iodo-3-(4-morpholinyl)rifamycin S (Compound C11)

By following the procedure of Example 7, but starting from Compound C8 (1.25 g) and NaI (0.5 g), the title compound is obtained (1.05 g).
TLC (methanol:dichloromethane, 1:9): black spot Rf 0.6

| Analysis for C₄₁H₅₁IN₂O₁₃, MW = 906.772 | | | |
|---|---|---|---|
| Calculated | C 54.31 | H 5.67 | N 3.09 |
| Found | C 53.82 | H 5.75 | N 2.79 |

### Example 9: 36-Diethylaminorifamycin S (Compound C12)

A mixture of Compound C1 (300 mg), diethylamine (100 mg) and tetrahydrofuran (5 ml) is stirred 4 hours at room temperature, then poured into water (25 ml) and extracted with ethyl acetate (3 x 15 ml). The combined extracts are dried and evaporated to dryness. The residue is purified by flash-chromatography; elution with 2% methanol in dichloromethane gives the pure title compound (135 mg).
TLC (methanol:dichloromethane, 1:9): Rf 0.5

| Analysis for C₄₁H₅₄N₂O₁₂, MW = 766.993 | | | |
|---|---|---|---|
| Calculated | C 64.20 | H 7.10 | N 3.65 |
| Found | C 63.64 | H 6.95 | N 3.38 |

### Example 10: 36-Acetyloxyrifamycin S (Compound C13)

A mixture of Compound C10 (180 mg), silver acetate (250 mg) and anhydrous dimethylformamide (18 ml) is stirred 18 hours at room temperature. The solvent is distilled off at 40°C under vacuum. The residue is purified by flash-chromatography; elution with 1.2% methanol in dichloromethane allowed the recovery of unreacted Compound C10 (66 mg) as less polar component and the isolation of the pure title compound (41 mg).
TLC (methanol:dichloromethane, 1:9): Rf 0.52

| Analysis for C₃₉H₄₇NO₁₄, MW = 753.809 | | | |
|---|---|---|---|
| Calculated | C 62.14 | H 6.28 | N 1.86 |
| Found | C 61.48 | H 6.40 | N 1.85 |

### Example 11: 36-Formyloxy-3-(4-morpholinyl)rifamycin S (Compound C14)

A mixture of potassium formate (11.5 g), dimethylformamide (800 ml) and activated 4Å molecular sieves (30g) is stirred for 20 minutes. Compound C8 (11.5 g) is then added in small portions and stirring continued for 24 hours at room temperature. The mixture is filtered and the solvent removed at 40°C under vacuum. The residue is purified by flash-chromatography; elution with 1.5% methanol in dichloromethane gives the pure title compound (8.6 g).
TLC (methanol:dichloromethane, 1:9): black spot Rf 0.42

| Analysis for C₄₂H₅₂N₂O₁₅, MW = 824.886 | | | |
|---|---|---|---|
| Calculated | C 61.15 | H 6.35 | N 3.40 |
| Found | C 60.41 | H 6.37 | N 3.30 |

### Example 12: 36-Hydroxy-3-(4-morpholinyl)rifamycin S (Compound C15)

A solution of potassium bicarbonate (18 g) in water (180 ml) is slowly added to Compound C14 (8.2 g) dissolved in methanol (450 ml). The resulting mixture is stirred overnight at room temperature, then evaporated to dryness at 30°C and under vacuum. Citric acid (10% ^{w}/ᵥ) is carefully added. The mixture is extracted with ethyl acetate (2 x 150 ml), which is then evaporated off. The residue is purified by flash-chromatography; elution with 1.8% methanol in dichloromethane, after crystallization from ethyl ether, gives the pure title compound (4.3 g). M.P, 168-171°C (dec).
TLC (methanol:dichloromethane, 5:95): black spot Rf 0.33

| Analysis for C₄₁H₅₂N₂O₁₄, MW = 796.876 | | | |
|---|---|---|---|
| Calculated | C 61.79 | H 6.58 | N 3.51 |
| Found | C 61.30 | H 6.56 | N 3.22 |

### Example 13: 36-Iodo-2'-diethylaminorifamycin P (Compound C16)

Sodium iodide (NaI) (0.2 g) dissolved in acetone (2 ml) is added to compound C9 (0.55 g) dissolved in acetone (4 ml). The resulting mixture is stirred at room temperature for 4 hours. The solvent is evaporated off and the residue purified by flash-chromatogarphy. Elution with 1.4% methanol in dichloromethane gives pure title compound (0.48 g).
TLC (methanol:dichloromethane, 1:9): orange spot Rf 0.48

| Analysis for C₄₂B₅₄IN₃O₁₁S, MW = 953.879 | | | | |
|---|---|---|---|---|
| Calculated | C 53.90 | H 5.82 | N 4.49 | S 3.42 |
| Found | C 54.04 | H 5.82 | N 4.48 | S 3.21 |

### Example 14: 36-Bromo-3-{[(4-methyl-1-piperazinyl)-imino]methyl}rifamycin SV (Compound C17)

a) N-methylene-t-butylamine (1.92 g) is slowly dropped into a cooled solution (15°C) of Compound C1 (3.3 g) in tetrahydrofuran (25 ml). After an addition of tert-butylamine (0.4 ml), the reaction mixture is stirred for 5 minutes, then manganese dioxide (1.7 g) is added and stirring continued overnight at 48°C. After cooling, the mixture is filtered and dropped into a cold solution (0°C) of 16% sulfuric acid (15 ml), ascorbic acid (3 g) and tetrahydrofuran (5 ml). The reaction mixture is stirred 3 hours at 45°C, then is poured in icy water (150ml) and finally extracted with ethyl acetate (3 x 5 ml) that is dried and evaporated to dryness. The residue is purified by flash-chromatography; elution with 3% methanol in dichloromethane gives pure 36-Bromo-3-formylrifamycin SV (500 mg).
TLC (methanol:dichloromethane, 85:15): red spot Rf 0.44

| Analysis for C₃₈H₄₆BrNO₁₃, HW = 804.699 | | | | |
|---|---|---|---|---|
| Calculated | C 56.72 | H 5.76 | N 1.74 | Br 9.93 |
| Found | C 56.06 | H 5.94 | N 2.00 | Br 10.10 |

b) 1-Amino-4-methylpiperazine (50 mg) is added to the above obtained compound (300 mg) dissolved in tetrahydrofuran (7 ml). The solution is stirred 30 min at room temperature. The solvent is evaporated off and the residue dissolved in ethyl acetate; addition of petroleum ether leads to the precipitation of the pure title compound (280 mg).
TLC (methanol:dichloromethane, 15:85): orange spot
Rf 0.65

| Analysis for C₄₃H₅₇BrN₄O₁₂, MW = 901.862 | | | | |
|---|---|---|---|---|
| Calculated | C 57.27 | H 6.37 | N 6.21 | Br 8.86 |
| Found | C 56.45 | H 6.40 | N 5.90 | Br 8.34 |

### Example 15: 36-Chloro-3-{[(4-methyl-1-piperazinyl)-imino]methyl]rifamycin SV (Compound C18)

a) By following the procedure of Example 14, step a, but starting from Compound C3 (4.1 g), N-methylene-t-butylamine (2.6 g), tert-butylamine (0.5 ml) and manganese dioxide (2.3 g), 36-chloro-3-formylrifamycin SV is obtained (3.9 g).
TLC (methanol:dichloromethane, 15:85): red spot Rf 0.47

| Analysis for C₃₈H₄₆ClNO₁₃, MW = 760.243 | | | | |
|---|---|---|---|---|
| Calculated | C 60.03 | H 6.10 | N 1.84 | Cl 4.66 |
| Found | C 59.61 | H 6.22 | N 1.80 | Cl 4.45 |

b) By following the procedure of Example 14, step b, but starting from the above obtained compound (3.9 g) and 1-amino-4-methylpiperazine (0.67 g), the title compound (2.92 g) is obtained.
TLC (methanol:dichloromethane, 15:85): orange spot
Rf 0.61

| Analysis for C₄₃H₅₇ClN₄O₁₂, MW = 857.406 | | | | |
|---|---|---|---|---|
| Calculated | C 60.23 | H 6.70 | N 6.53 | Cl 4.13 |
| Found | C 59.94 | H 6.42 | N 6.36 | Cl 4.40 |

### Example 16: 36-Iodo-3-{[(4-methyl-1-piperazinyl)-imino]methyl}rifamycin SV (Compound C19)

Sodium iodide (110 mg) is added to Compound C17 (300 mg) dissolved in acetone (3 ml). The mixture is stirred 4 hours at room temperature and then evaporated to dryness. The residue is purified by flash-chromatography; elution with 3% methanol in dichloromethane gives pure title compound (160 mg).

Alternatively, Compound C18 (2.9 g) is reacted with sodium iodide (1 g) as above described, thus obtaining the pure title compound (2.83 g).
TLC (methanol:dichloromethane, 15:85): orange spot
Rf 0.63

| Analysis for C₄₃H₅₇IN₄O₁₂, MW = 948.857 | | | |
|---|---|---|---|
| Calculated | C 54.43 | H 6.05 | N 5.90 |
| Found | C 54.70 | H 6.20 | N 5.49 |

### Example 17: 36-Bromo-3-{[(4-cyclopentyl-1-piperazinyl)imino]methyl}rifamycin SV (Compound C20)

By following the procedure of Example 14, step b, but starting from 36-Bromo-3-formylrifamycin SV (0.65 g) and 1-Amino-4-cyclopentylpiperazine (0.14 g), the title compound is obtained (0.64 g).
TLC (methanol:dichloromethane, 15:85): orange spot
Rf 0.64

| Analysis for C₄₇H₆₃BrN₄O₁₂, MW = 955.955 | | | | |
|---|---|---|---|---|
| Calculated | C 59.05 | H 6.64 | N 5.86 | Br 8.36 |
| Found | C 58.38 | H 6.39 | N 5.60 | Br 7.61 |

### Example 18: 36-Iodo-3-{[(4-cyclopentyl-1-piperazinyl)imino]methyl}rifamycin SV (Compound C21)

By following the procedure of Example 16, but starting from Compound C20 (730 mg) and sodium iodide (250 mg), the title compound (432 mg) is obtained.

### Example 19: 36-[(1-Ethyl-6-fluoro-1,4-dihydro-7-(4-methyl-1-piperazinyl)-4-oxo-3-quinolinyl)carbonyloxy]-2'-(diethylamino)rifamycin P (Compound C22)

(1-Ethyl-6-fluoro-1,4-dihydro-7-(4-methyl-1-piperazinyl)-4-oxo-3-quinolinyl)carboxylic acid (Pefloxacin) potassium salt (440 mg) dissolved in dimethylformamide (44 ml) is stirred for 30 min with activated 4Å molecular sieves (Union Carbide type 4Å, Fluka) (4.4 g). Compound C16 (440 mg) is added in small portions and stirring continued overnight at room temperature. The reaction mixture is filtered and evaporated to dryness at 40°C under vacuum. The residue is purified by flash-chromatography; elution with 8% methanol in dichloromethane allows the isolation of the pure title compound (380 mg).
TLC (methanol:dichloromethane, 1: 9): orange spot Rf 0.4

| Analysis for C₅₉H₇₃FN₆O₁₄S, MW = 1141.313 | | | | |
|---|---|---|---|---|
| Calculated | C 62.09 | H 6.45 | N 7.36 | S 2.81 |
| Found | C 61.20 | H 6.36 | N 7.05 | S 2.44 |

### Example 20: 36-{2-[1-Ethyl-6-fluoro-1,4-dihydro-7-(4-methyl-1-piperazinyl)-4-oxo-3-quinolinyl]carbonyloxy} rifamycin S (Compound C23)

Pefloxacin potassium salt (250 mg) is reacted with Compound C10 (250 mg) in dimethylformaaide (25 ml) as described in Example 19. In this way the title compound (178 mg) is obtained.
TLC (methanol:dichloromethane, 1:9): brown spot Rf 0.38

| Analysis for C₅₄H₆₃FN₄O₁₅, MW = 1027.120 | | | |
|---|---|---|---|
| Calculated | C 63.14 | H 6.18 | N 5.45 |
| Found | C 62.50 | H 5.98 | N 5.06 |

### Example 21: 36-{[1-Ethyl-6-fluoro-1,4-dihydro-7-(4-methyl-1-piperazinyl)-4-oxo-3-quinolinyl]carbonyloxy}-3-(4-morpholinyl) rifamycin S (Compound C24)

Compound C8 (460 mg) is reacted with Pefloxacin potassium salt (460 mg) in dimethylformamide (46 ml) as described in Example 19. The residue is purified by flash-chromatography; elution with 6% methanol in dichloromethane gives pure title compound (354 mg).
TLC (methanol:dichloromethane, 1:9): black spot Rf 0.42

| Analysis for C₅₈H₇₀EN₅O₁₆, MW = 1112.225 | | | |
|---|---|---|---|
| Calculated | C 62.63 | H 6.34 | N 6.29 |
| Found | C 61.72 | H 6.37 | N 5.95 |

### Example 22: 36-{[1-Ethyl-6-fluoro-1,4-dihydro-7-(4-methyl-1-piperazinyl)-4-oxo-3-quinolinyl]carbonyloxy}-3-{[(4-methyl-1-piperazinyl)imino]methyl}rifamycin SV (Compound C25)

By reacting Pefloxacin potassium salt (650 mg) with Compound C19 (650 mg) in dimethylformamide (65 ml), as described in Example 19, the title compound (480 mg) is obtained after purifying twice by flash-chromatography (elution with increasing percentages - 4 to 15% - of methanol in dichloromethane).
TLC (methanol:dichloromethane, 2:8): orange spot Rf 0.47

| Analysis for C₆₀H₇₆FN₇O₁₅, MW = 1154.310 | | | |
|---|---|---|---|
| Calculated | C 62.43 | H 6.63 | N 8.49 |
| Found | C 62.06 | H 6.18 | N 8.17 |

### Example 23: 3-[[(4-Cyclopentyl-1-piperazinyl)-imino]methyl}-36-{[1-ethyl-6-fluoro-1,4-dihydro-7-(4-methyl-1-piperazinyl)-4-oxo-3-quinolinyl]carbonyloxy}-rifamycin SV (Compound C26)

By following the procedure described in Example 22, but starting from the Compound C21 (400 mg) and Pefloxacin potassium salt (400 mg), the title compound (280 mg) is obtained.
TLC (methanol:dichloromethane, 2:8): orange spot Rf 0.60

| Analysis for C₆₄H₈₂FN₇O₁₅, MW = 1208.403 | | | |
|---|---|---|---|
| Calculated | C 63.61 | H 6.84 | N 8.11 |
| Found | C 63.70 | H 1.00 | N 8.02 |

### Example 24: 36-{[1-Ethyl-1,4-dihydro-1-methyl-4-oxo-1,8-naphthyridin-3-yl)carbonyloxy}-3-{[(4-methyl-1-piperazinyl)imino]methyl}rifamycin SV (Compound C27)

According to the procedure of Example 22, but starting from Compound C19 (500 mg) and [1-Ethyl-1,4-dihydro-7-methyl-4-oxo-1,8-naphthyridin-3-yl)carboxylic acid (Nalidixic Acid) potassium salt (500 mg), the title compound (452 mg) is obtained.
TLC (methanol:dichloromethane, 1:9): red-orange spot
Rf 0.49

| Analysis for C₃₅H₆₈N₆O₁₅, MW = 1053.186 | | | |
|---|---|---|---|
| Calculated | C 62.72 | H 6.51 | N 7.98 |
| Found | C 61.86 | H 6.43 | N 7.79 |

### Example 25: 36-{[8-Ethyl-5,8-dihydro-2-(4-methyl-1-piperazinyl)-5-oxopyrido[2,3-d]pyrimidin-6-yl]carbonyloxy}-3-{[(4-methyl-1-piperazinyl)imino]-methyl}rifamycin SV (Compound C28)

According to Example 22, but starting from N-methyl-pipemidic acid potassium salt (400 mg) and Compound C19 (400 mg), the pure title compound (275 mg) is obtained.
TLC (methanol:dichloromethane, 15:85): red-orange spot
Rf 0.36

| Analysis for C₅₈H₇₅N₉O₁₅, MW = 1138.295 | | | |
|---|---|---|---|
| Calculated | C 61.20 | H 6.64 | N 11.07 |
| Found | C 60.89 | H 6.60 | N 10.71 |

### Example 26: 36-{[1-Cyclopropyl-6-fluoro-1,4-dihydre-7-(2,6-dimethyl-4-pyridinyl)-4-oxo-3-quinolinyl]-carbonyloxy}-3-{[(4-methyl-1-piperazinyl)imino]-methyl}rifamycin SV (Compound C29)

According to Example 22, but starting from Compound P7 (500 mg) and Compound C19 (500 mg), the pure title compound (420 mg) is obtained.
TLC (methanol:dichloromethane, 1:9): red-orange spot
Rf 0.45

| Analysis for C₆₃H₇₃FN₆O₁₅, MW = 1173.313 | | | |
|---|---|---|---|
| Calculated | C 64.49 | H 6.27 | N 7.16 |
| Found | C 64.47 | H 6.38 | N 7.10 |

### Example 27: 36-{[1-Ethyl-1,4-dihydro-6-(4-methyl-1-piperazinyl)-4-oxo-3-pyridinyl]carbonyloxy]-3-{[(4-methyl-1-piperazinyl)imino]methyl}rifamycin SV (Compound C30)

According to Example 22, but starting from Compound P6 (500 mg) and Compound C19 (500 mg), the pure title compound (320 mg) is obtained.
TLC (methanol:dichloromethane, 15:85): red-orange spot
Rf 0.44

| Analysis for C₅₆H₇₅N₇O₁₅, MW = 1086.260 | | | |
|---|---|---|---|
| Calculated | C 61.92 | H 6.96 | N 9.02 |
| Found | C 62.00 | H 6.96 | N 8.91 |

## Claims

1. Compound of general formula I: 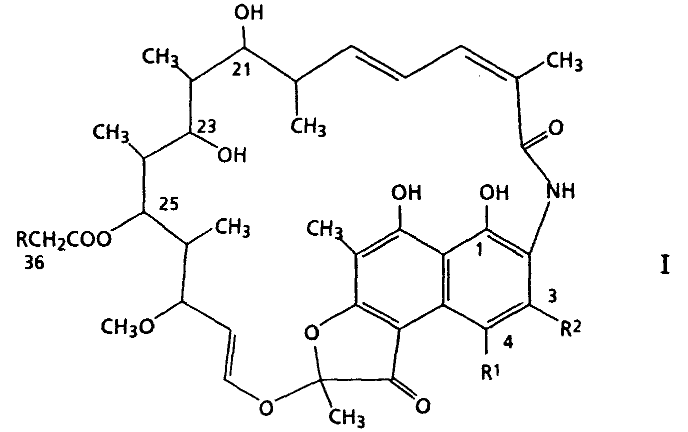 or the oxidated derivatives thereof of formula Ia: 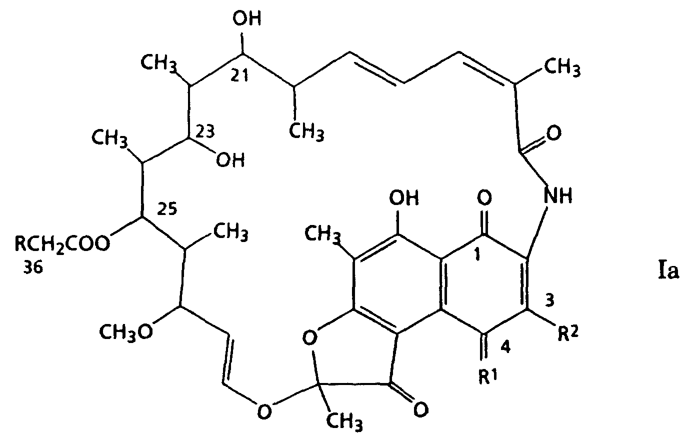 wherein:
R represents halo, hydroxy, thio, (C₁-C₄)alkoxy, (C₁-C₄)alkylthio, (C₁-C₄)acyloxy, (C₁-C₄)alkylamino, di(C₁-C₄)alkylamino or a group of formula: 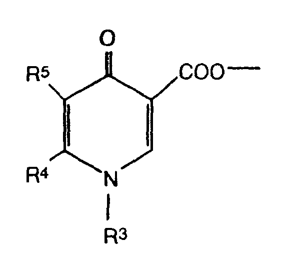 wherein:
R³ represents (C₁-C₄)alkyl or (C₃-C₆)cycloalkyl;
R⁴ represents a group of formula 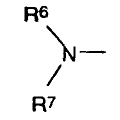 wherein:
R⁶ and R⁷ independently represent hydrogen or (C₁-C₄)alkyl or
R⁶ and R⁷ together with the adjacent nitrogen atom form a five or six membered heterocyclic ring, optionally containing one further heteroatom selected from oxygen, nitrogen and sulfur, wherein one of the carbon or nitrogen atoms of the ring is optionally substituted by a (C₁-C₄)alkyl moiety;
R⁵ is hydrogen or halogen;
or R⁴ together with R⁵ form a bifunctional alkylenic chain, optionally containing 1 or 2 nitrogen atoms, of the following formula: 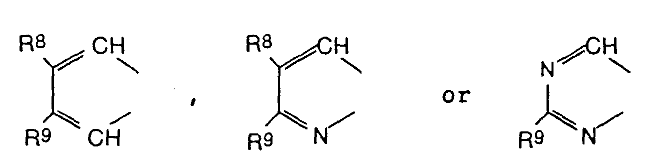 wherein:
R⁸ represents hydrogen or halogen;
R⁹ represents (C₁-C₄)alkyl, or a six membered heterocyclic ring containing one or two nitrogen atoms, wherein the carbon and nitrogen atoms of the ring are optionally substituted with one or two (C₁-C₄)alkyl moieties;
R¹ represents hydroxy in formula I or oxygen in formula Ia;
R² represents hydrogen, a five or six membered heterocyclic ring containing one or two heteroatoms selected from oxygen, nitrogen and sulfur, wherein one of the carbon or nitrogen atoms of the ring is optionally substituted by a (C₁-C₄)alkyl moiety, or a group of formula:
-CH=N-R¹⁰
wherein R¹⁰ represents a six membered heterocyclic ring containing one or two nitrogen atoms, wherein one of the carbon or nitrogen atoms of the ring is optionally substituted by (C₁-C₄)-alkyl or (C₅-C₆)cycloalkyl;
or R¹ and R² taken together form a group of formula
=N-(CHR¹¹)-X-, -NH-(CHR¹¹)-X-, or -N=(CR¹¹)-X-,
wherein:
X represents a sulfur atom or a -NH- group and
R¹¹ represents hydrogen, (C₁-C₄)alkyl, (C₁-C₄)alkylamino or di(C₁-C₄)alkylamino;
and the pharmaceutically acceptable base addition salts thereof.

2. Compound according to claim 1 wherein:
R is halo, hydroxy, (C₁-C₄)acyloxy, (C₁-C₄)alkoxy, (C₁-C₄)alkylthio, di(C₁-C₄)alkylamino or a group of formula: 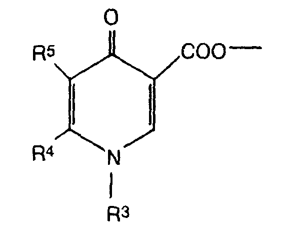 wherein:
R³ represents (C₁-C₄)alkyl or (C₃-C₆)cycloalkyl;
R⁴ represents a group of formula 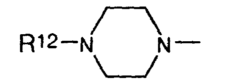 wherein R¹² represents hydrogen or (C₁-C₄)alkyl;
R⁵ is hydrogen or halo;
or R⁴ together with R⁵ form a bifunctional alkylenic chain, optionally containing 1 or 2 nitrogen atoms, of the following formula: 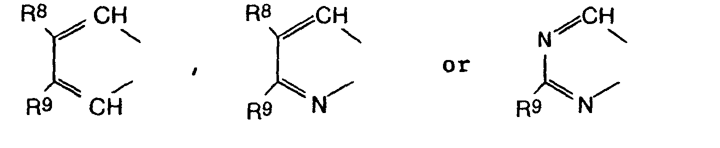
wherein R⁹ represents (C₁-C₄)alkyl, a group of formula 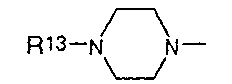 wherein R¹³ is hydrogen or (C₁-C₄)alkyl, or a group of formula 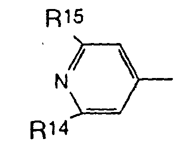 wherein R¹⁴ and R¹⁵ independently represent hydrogen or (C₁-C₄)alkyl;
R¹ is hydroxy in the reduced form or oxygen in the oxidated form;
R² represents hydrogen, a six membered heterocyclic ring containing one or two heteroatoms selected from oxygen, nitrogen and sulfur, wherein one of the carbon or nitrogen atoms of the ring is optionally substituted by a (C₁-C₄)alkyl moiety, or a group of formula: 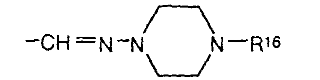 wherein R¹⁶ represents (C₁-C₄)alkyl or (C₅-C₆)cycloalkyl;
or R¹ and R² taken together form a group of formula -N=CR¹¹-S- wherein R¹¹ represents hydrogen, (C₁-C₄)alkyl or di(C₁-C₄)alkylamino.

3. Compound according to claim 1 wherein:
R is fluoro, bromo, chloro, iodo, hydroxy, formyloxy, acetyloxy, thiomethyl, diethylamino or a group of formula: 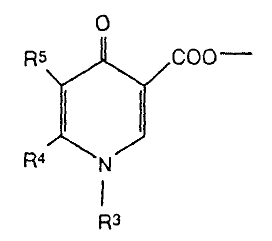 wherein:
R³ is ethyl or cyclopropyl, R⁴ is 4-methyl-1-piperazinyl and R⁵ is hydrogen;
or R⁴ together with R⁵ form a bifunctional alkylenic chain, optionally containing 1 or 2 nitrogen atoms, of formula: 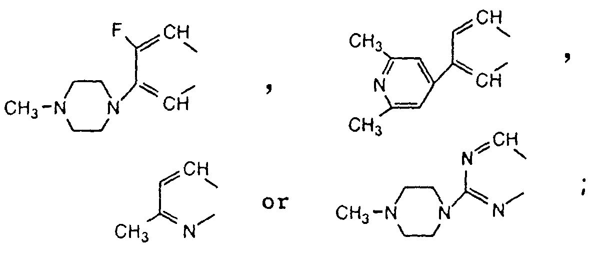
R¹ is hydroxy in the reduced form or oxygen in the oxidated form;
R² is hydrogen, 4-morpholinyl, {[(4-methyl-1-piperazinyl)imino]methyl} or {[(4-cyclopentyl-1-piperazinyl)imino]methyl};
or R¹ and R² together form a group of formula

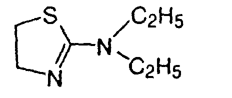

4. Compound according to claim 1 wherein:
R is bromo, chloro, iodo, hydroxy or a group of formula: 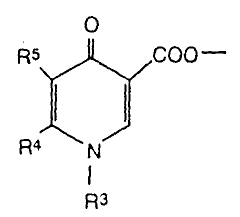 wherein:
R³ is ethyl, R⁴ is 4-methyl-1-piperazinyl and R⁵ is hydrogen;
or R⁴ together with R⁵ form a bifunctional alkylenic chain of formula: 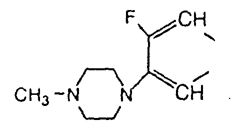
R¹ is hydroxy in the reduced form or oxygen in the oxidated form and R² is hydrogen, 4-morpholinyl or {[(4-methyl-1-piperazinyl)-imino]methyl}.

5. Compound according to claim 1, 2, 3 or 4 wherein the pharmaceutically acceptable salts of the compounds of formula I are formed with alkali metal, earth-alkali metal, (C₁-C₄)alkylamines, (C₁-C₄)alkanolamines or basic aminoacids.

6. Compound of general formula 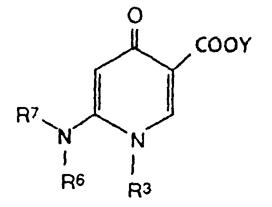 wherein:
Y represents hydrogen or (C₁-C₄)alkyl and R³, R⁶ and R⁷ are as defined in claim 1,
and the alkali metal salts thereof.

7. Process for preparing a compound of formula I wherein R, R¹ and R² are as defined in claim 1, which comprises:
a) reacting a compound of general formula II 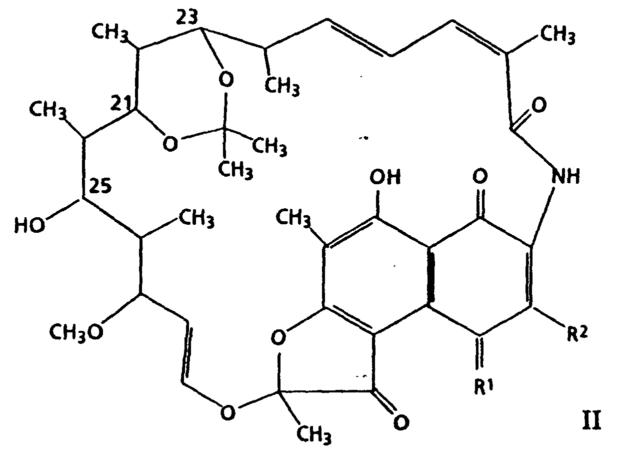 wherein R¹ and R² have the same meanings as in claim 1, with the proviso that R² is not a group of formula
-CH=N-R¹⁰,
with a malonic acid derivative of general formula III: 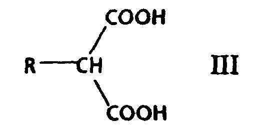 wherein R is as above defined, in the presence of a condensing agent and an inert organic solvent;
b) removing the protection in positions 21 and 23 by means of an acidic cleavage of the acetonidic moiety in the presence of an inert organic solvent; and
c) contacting the obtained deprotected intermediate compound with a cuprous salt or oxide or a mixture thereof in the presence of an inert organic solvent.

8. Process according to claim 7 wherein the inert organic solvents of steps a, b and c are selected from alkylamides, alkylnitriles, saturated linear or cyclic ethers, glycol ethers, phosphoramides, sulfoxides, chlorinated solvents and mixtures thereof.

9. Process according to claim 7 wherein the condensing agent of step a is selected from carboxydiimides, dialkylaminopyridines, carbonylimidazoles, triphenylphosphine, substituted dithiocarbonates and diphenylphosphorylazides.

10. Process according to claim 7 wherein the acidic cleavage of the acetodinic moiety in step b is performed under mild acidic conditions by means of a diluted mineral or organic sulfonic acid.

11. Process according to claim 7 wherein the decarboxylating agent of step c is selected from Cu₂O, Cu₂S, CuCl, CuBr and Cu₂SO₄ or a mixture thereof.

12. Process according to anyone of the claims from 8 to 11 wherein the reaction temperature of step a is from 0°C to 35°C, the reaction temperature of step b is from 30°C to 50°C and the reaction temperature of step c is conducted at from 50°C to 75°C.

13. Process for preparing a compound of general formula I, wherein R is as defined in claim 1, R¹ is oxygen and R² is a group -CH=N-R¹⁰, wherein R¹⁰ is as defined in claim 1, which comprises contacting a compound of general formula I, wherein R and R¹ are as above defined and R² is hydrogen with N-methylene-t-butylamine in the presence of t-butylamine and manganese dioxide and then with a compound of formula NH₂-R¹⁰, wherein R¹⁰ is as above defined.

14. Process for preparing a compound of general formula I, wherein R is iodo and R¹ and R² are as defined in claim 1, which comprises contacting a compound of general formula I, wherein R is chloro or bromo and R¹ and R² are as defined in claim 1, with an acetone solution of an alkali metal iodide.

15. Process for preparing a compound of general formula I, wherein R is (C₁-C₄)acyloxy, (C₁-C₄)alkylamino, di(C₁-C₄)alkylamino or a group of formula 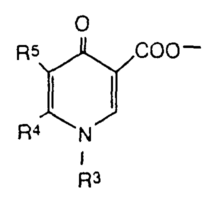 as defined in claim 1, R¹ and R² being as defined in claim 1, which comprises reacting a compound of general formula I, wherein R is chloro, bromo or iodo and R¹ and R² are as defined in claim 1, respectively with a (C₁-C₄)acylate salt, a (C₁-C₄)alkylamine, a di(C₁-C₄)alkylamine, or a salt of a 4-oxo-3-pyridinyl carboxylic acid derivative of general formula IV 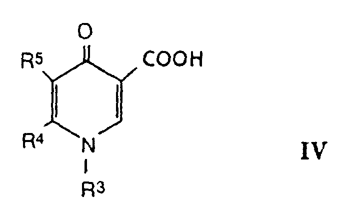 wherein R³, R⁴ and R⁵ are as above defined, in the presence of an inert organic solvent.

16. Process according to claim 15 wherein the (C₁-C₄)acylate salt or the salt of the 4-oxo-3-pyridinyl carboxylic acid derivative is an alkali metal or a silver salt.

17. Process for preparing a compound of general formula I, wherein R is hydroxy and R¹ and R² are as defined in claim 1, which comprises hydrolyzing a compound of general formula I, wherein R is formyloxy and R¹ and R² are as defined in claim 1, under mild basic conditions in an hydroalcoholic solution.

18. Compound according to anyone of claim 1, 2, 3, 4 or 5 for use as medicament.

19. Pharmaceutical composition containing as an active ingredient a compound of claim 1, 2, 3, 4 or 5 in admixture with a pharmaceutically acceptable carrier.

20. Use of a compound of claim 1, 2, 3, 4 or 5 for preparing a medicament for treating infections related to the presence of microorganisms susceptible to it.

21. A compound of claim 1, 2, 3, 4 or 5 for use as animal growth promoter.

22. Use of a compound of claim 1, 2, 3, 4 or 5 for preparing a composition for use as animal growth promoter.

## Patentansprüche

1. Verbindung der allgemeinen Formel I: 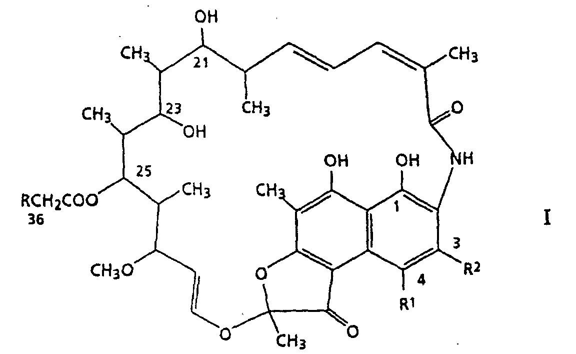 oder deren oxidierte Derivate der Formel Ia: 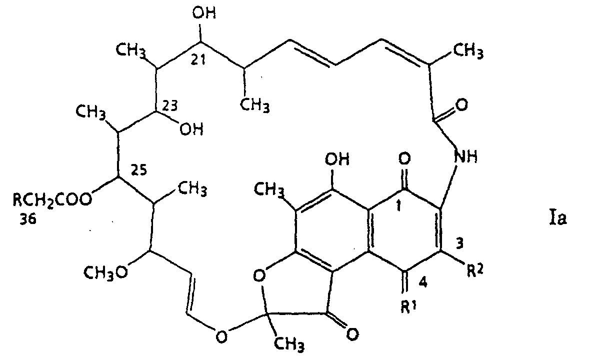 wobei R ein Halogenatom, eine Hydroxy-, Thiogruppe, einen (C₁-C₄)Alkoxy-, (C₁-C₄)Alkylthio-, (C₁-C₄)Acyloxy-, (C₁-C₄)Alkylamino, Di(C₁-C₄)alkylaminorest oder einen Rest der Formel: 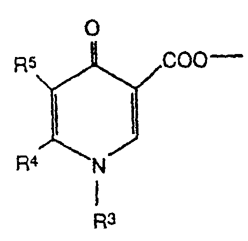 darstellt, wobei:
R³ einen (C₁-C₄)-Alkyl- oder (C₃-C₆)-Cycloalkylrest darstellt;
R⁴ einen Rest der Formel 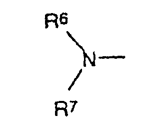 darstellt, wobei:
R⁶ und R⁷ unabhängig voneinander ein Wasserstoffatom oder einen (C₁-C₄)Alkylrest darstellen oder
R⁶ und R⁷ mit dem benachbarten Stickstoffatom einen fünf- oder sechsgliedrigen heterocyclischen Ring bilden, der gegebenenfalls ein weiteres, aus einem Sauerstoff-, Stickstoff- und Schwefelatom ausgewähltes Heteroatom enthält, wobei eines der Kohlenstoff- oder Stickstoffatome des Rings gegebenenfalls mit einer (C₁-C₄)Alkyleinheit substituiert ist;
R⁵ ein Wasserstoff- oder Halogenatom ist;
oder R⁴ und R⁵ zusammen eine bifunktionelle Alkylenkette, die gegebenenfalls 1 oder 2 Stickstoffatome enthält, der nachstehenden Formel bilden: 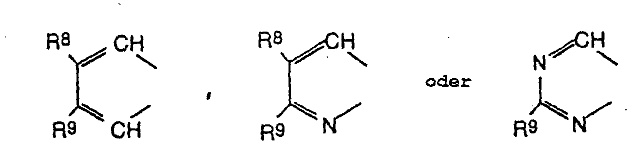 wobei R⁸ ein Wasserstoff- oder Halogenatom darstellt;
R⁹ einen (C₁-C₄)-Alkylrest oder einen sechsgliedrigen heterocyclischen Ring darstellt, der ein oder zwei Stickstoffatome enthält, wobei die Kohlenstoff- und Stickstoffatome des Rings gegebenenfalls mit einer oder zwei (C₁-C₄)Alkyleinheiten substituiert sind;
R¹ in Formel I eine Hydroxygruppe oder in Formel Ia ein Sauerstoffatom darstellt;
R² ein Wasserstoffatom, einen fünf- oder sechsgliedrigen heterocyclischen Ring mit einem oder zwei aus Sauerstoff-, Stickstoff- und Schwefelatomen ausgewählten Heteroatomen, wobei eines der Kohlenstoff- oder Stickstoffatome des Rings gegebenenfalls durch eine (C₁-C₄)Alkyleinheit substituiert ist, oder einen Rest der Formel:
-CH=N-R¹⁰
darstellt, wobei R¹⁰ einen sechsgliedrigen heterocyclischen Ring mit einem oder zwei Stickstoffatomen darstellt, wobei eines der Kohlenstoff- oder Stickstoffatome des Rings gegebenenfalls durch einen (C₁-C₄)Alkyl- oder (C₅-C₆)Cycloalkylrest substituiert ist;
oder R¹ und R² zusammen einen Rest der Formel =N-(CHR¹¹)-X-, -NH-(CHR¹¹)-X-oder -N=(CR¹¹)-X- bilden,
wobei:
X ein Schwefelatom oder eine -NH-Gruppe darstellt und
R¹¹ ein Wasserstoffatom, einen (C₁-C₄)Alkyl-, (C₁-C₄)Alkylamino- oder Di(C₁-C₄)alkylaminorest darstellt;
und die pharmazeutisch verträglichen Basenadditionssalze davon.

2. Verbindung nach Anspruch 1, wobei:
R ein Halogenatom, eine Hydroxygruppe, ein (C₁-C₄)Acyloxy-, (C₁-C₄)Alkoxy, (C₁-C₄)Alkylthio-, Di(C₁-C₄)alkylaminorest oder ein Rest der Formel: 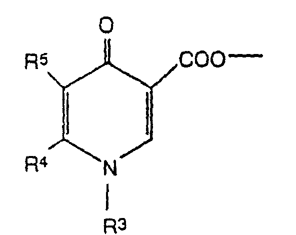 ist, wobei:
R³ einen (C₁-C₄)Alkyl- oder (C₃-C₆)Cycloalkylrest darstellt;
R⁴ einen Rest der Formel 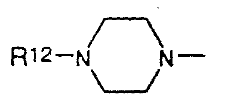 darstellt, wobei R¹² ein Wasserstoffatom oder einen (C₁-C₄)Alkylrest darstellt;
R⁵ ein Wasserstoffatom oder Halogenatom darstellt;
oder R⁴ und R⁵ zusammen eine bifunktionelle Alkylenkette, die gegebenenfalls 1 oder 2 Stickstoffatome enthält, der nachstehenden Formel: 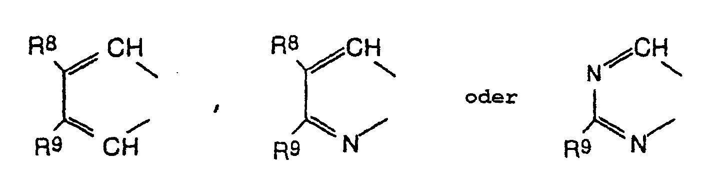 bilden, wobei R⁹ einen (C₁-C₄)Alkylrest, einen Rest der Formel 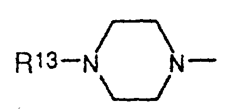
wobei R¹³ ein Wasserstoffatom oder einen (C₁-C₄)Alkylrest bedeutet, oder einen Rest der Formel 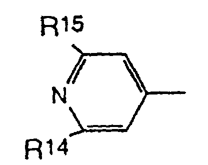 darstellt, wobei R¹⁴ und R¹⁵ unabhängig voneinander ein Wasserstoffatom oder einen (C₁-C₄)Alkylrest darstellen;
R¹ in der reduzierten Form eine Hydroxygruppe oder in der oxidierten Form ein Sauerstoffatom ist;
R² ein Wasserstoffatom, einen sechsgliedrigen heterocyclischen Ring mit einem oder zwei aus Sauerstoff-, Stickstoff- und Schwefelatomen ausgewählten Heteroatomen, wobei eines der Kohlenstoff- oder Stickstoffatome des Rings gegebenenfalls durch eine (C₁-C₄)-Alkyleinheit substituiert ist, oder einen Rest der Formel: 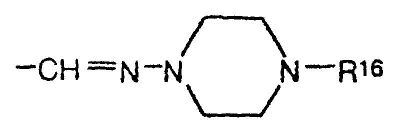 darstellt, wobei R¹⁶ einen (C₁-C₄)Alkyl- oder (C₅-C₆)Cycloalkylrest darstellt;
oder R¹ und R² zusammen einen Rest der Formel -N=CR¹¹-S- bilden, wobei R¹¹ ein Wasserstoffatom, einen (C₁-C₄)-Alkyl- oder Di(C₁-C₄)alkylaminorest darstellt.

3. Verbindung nach Anspruch 1, wobei:
R ein Fluor-, Brom-, Chlor-, Iodatom, eine Hydroxy-, Formyloxy-, Acetyloxy-, Thiomethyl-, Diethylaminogruppe oder ein Rest der Formel: 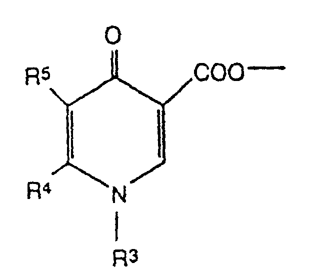 ist, wobei:
R³ eine Ethyl- oder Cyclopropylgruppe ist, R⁴ eine 4-Methyl-1-piperazinylgruppe ist und R⁵ ein Wasserstoffatom ist;
oder R⁴ und R⁵ zusammen eine bifunktionelle Alkylenkette, die gegebenenfalls 1 oder 2 Stickstoffatome enthält, der Formel 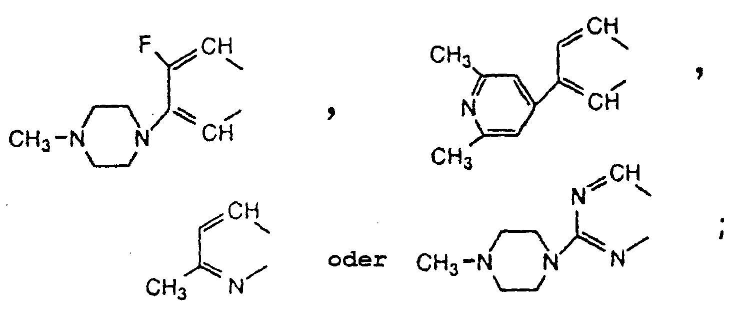 bilden,
R¹ in der reduzierten Form eine Hydroxygruppe ist oder in der oxidierten Form ein Sauerstoffatom ist.;
R² ein Wasserstoffatom, eine 4-Morpholinyl-, {[(4-Methyl-1-piperazinyl)imino]methyl}-oder {[(4-Cyclopentyl-1-piperazinyl)imino]methyl}gruppe ist;
oder R¹ und R² zusammen einen Rest der Formel 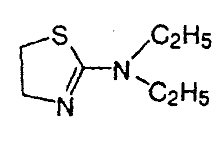 bilden.

4. Verbindung nach Anspruch 1, wobei:
R ein Brom-, Chlor-, Iodatom, eine Hydroxygruppe oder ein Rest der Formel: 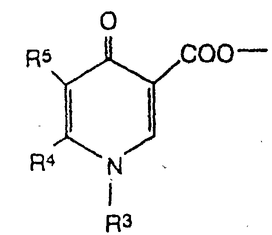 ist, wobei:
R³ eine Ethylgruppe ist, R⁴ eine 4-Methyl-1-piperazinylgruppe ist und R⁵ ein Wasserstoffatom ist;
oder R⁴ und R⁵ zusammen eine bifunktionelle Alkylenkette der Formel: 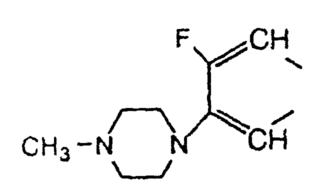 bilden;
R¹ in der reduzierten Form eine Hydroxygruppe oder in der oxidierten Gruppe ein Sauerstoffatom ist, und R² ein Wasserstoffatom, eine 4-Morpholinyl- oder {[(4-Methyl-1-piperazinyl)-imino]methyl}gruppe ist.

5. Verbindung nach Anspruch 1, 2, 3 oder 4, wobei die pharmazeutisch verträglichen Salze der Verbindungen der Formel I mit einem Alkalimetall, einem Erdalkalimetall, (C₁-C₄)Alkylaminen, (C₁-C₄)Alkanolaminen oder basischen Aminosäuren hergestellt werden.

6. Verbindung der allgemeinen Formel 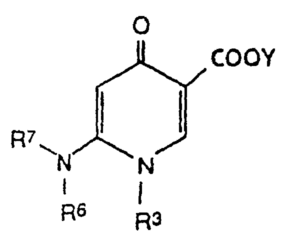 wobei
Y ein Wasserstoffatom oder einen (C₁-C₄)Alkylrest darstellt, und R³, R⁶ und R⁷ die in Anspruch 1 definierte Bedeutung haben,
und die Alkalimetallsalze davon.

7. Verfahren zur Herstellung einer Verbindung der Formel I, wobei R, R¹ und R² die in Anspruch 1 definierte Bedeutung haben, umfassend:
a) das Umsetzen einer Verbindung der allgemeinen Formel II 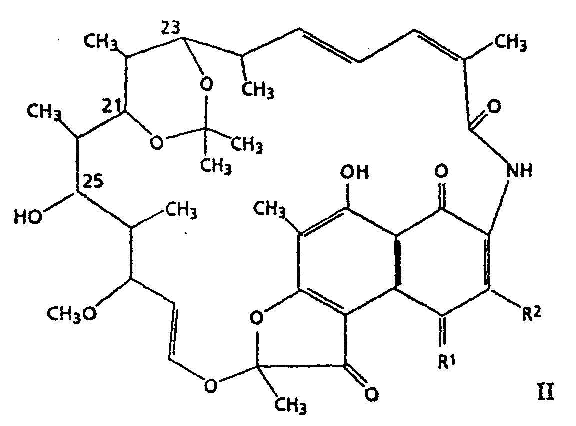 wobei R¹ und R² die in Anspruch 1 definierten Bedeutungen haben, mit der Maßgabe, dass R² kein Rest der Formel
-CH=N-R¹⁰ ist,
mit einem Malonsäurederivat der allgemeinen Formel III: 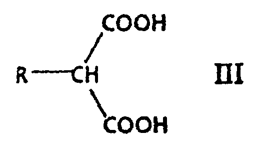 wobei R die vorstehend definierte Bedeutung hat, in Gegenwart eines Kondensationsmittels und eines inerten organischen Lösungsmittels;
b) das Entfernen der Schutzgruppen von den Positionen 21 und 23 durch Säurespaltung der Acetonideinheit in Gegenwart eines inerten organischen Lösungsmittels; und
c) das Inkontaktbringen der erhaltenen Zwischenverbindung ohne Schutzgruppen mit einem Kupfer(I)salz oder -oxid oder einem Gemisch davon in Gegenwart eines inerten organischen Lösungsmittels.

8. Verfahren nach Anspruch 7, wobei die inerten organischen Lösungsmittel der Schritte a, b und c aus Alkylamiden, Alkylnitrilen, gesättigten linearen oder cyclischen Ethern, Glycolethern, Phosphoramiden, Sulfoxiden, chlorierten Lösungsmitteln und Gemischen davon ausgewählt sind.

9. Verfahren nach Anspruch 7, wobei das Kondensationsmittel von Schritt a aus Carboxydiimiden, Dialkylaminopyridinen, Carbonylimidazolen, Triphenylphosphin, substituierten Dithiocarbonaten und Diphenylphosphorylaziden ausgewählt ist.

10. Verfahren nach Anspruch 7, wobei die Säurespaltung der Acetonideinheit in Schritt b unter milden sauren Bedingungen mit Hilfe einer verdünnten Mineral- oder organischen Sulfonsäure erfolgt.

11. Verfahren nach Anspruch 7, wobei das Decarboxylierungsmittel von Schritt c aus Cu₂O, Cu₂S, CuCl, CuBr und Cu₂SO₄ oder einem Gemisch davon ausgewählt ist.

12. Verfahren nach einem der Ansprüche 8 bis 11, wobei die Reaktionstemperatur von Schritt a von 0°C bis 35°C reicht, die Reaktionstemperatur von Schritt b von 30°C bis 50°C reicht und die Umsetzung von Schritt c bei einer Temperatur von 50°C bis 75°C erfolgt.

13. Verfahren zur Herstellung einer Verbindung der allgemeinen Formel I, wobei R die in Anspruch 1 definierte Bedeutung hat, R¹ ein Sauerstoffatom ist und R² ein Rest -CH=N-R¹⁰ ist, wobei R¹⁰ die in Anspruch 1 definierte Bedeutung hat, umfassend das Inkontaktbringen einer Verbindung der allgemeinen Formel I, wobei R und R¹ die vorstehend definierte Bedeutung haben und R² ein Wasserstoffatom ist, mit N-Methylen-t-butylamin in Gegenwart von t-Butylamin und Mangandioxid und dann mit einer Verbindung der Formel NH₂-R¹⁰, wobei R¹⁰ die vorstehend definierte Bedeutung hat.

14. Verfahren zur Herstellung einer Verbindung der allgemeinen Formel I, wobei R Iod ist und R¹ und R² die in Anspruch 1 definierte Bedeutung haben, umfassend das Inkontaktbringen einer Verbindung der allgemeinen Formel I, wobei R ein Chlor- oder Bromatom ist und R¹ und R² die in Anspruch 1 definierte Bedeutung haben, mit einer Aceton-Lösung eines Alkalimetalliodids.

15. Verfahren zur Herstellung einer Verbindung der allgemeinen Formel I, wobei R ein (C₁-C₄)Acyloxy-, (C₁-C₄)Alkylamino-, Di(C₁-C₄)alkylaminorest oder ein Rest der Formel 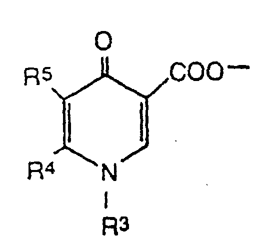 ist, wie in Anspruch 1 definiert, wobei R¹ und R² die in Anspruch 1 definierte Bedeutung haben, umfassend das Umsetzen einer Verbindung der allgemeinen Formel I, wobei R ein Chlor-, Brom- oder Iodatom ist, und R¹ und R² die in Anspruch 1 definierte Bedeutung haben, mit einem (C₁-C₄)Acylatsalz, einem (C₁-C₄)alkylamin, einem Di(C₁-C₄)alkylamin oder einem Salz eines 4-Oxo-3-pyridinylcarbonsäurederivates der allgemeinen Formel IV 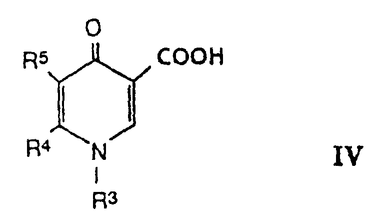 wobei R³, R⁴ und R⁵ die vorstehend definierte Bedeutung haben, in Gegenwart eines inerten organischen Lösungsmittels.

16. Verfahren nach Anspruch 15, wobei das (C₁-C₄)-Acylatsalz oder das Salz des 4-Oxo-3-pyridinylcarbonsäurederivates ein Alkalimetall- oder Silbersalz ist.

17. Verfahren zur Herstellung einer Verbindung der allgemeinen Formel I, wobei R eine Hydroxygruppe ist und R¹ und R² die in Anspruch 1 definierte Bedeutung haben, umfassend das Hydrolysieren einer Verbindung der allgemeinen Formel I, wobei R eine Formyloxygruppe ist, und R¹ und R² die in Anspruch 1 definierte Bedeutung haben, unter milden basischen Bedingungen in einer Hydroalkohollösung.

18. Verbindung nach einem der Ansprüche 1, 2, 3, 4 oder 5 zur Verwendung als Medikament.

19. Arzneimittel, enthaltend eine Verbindung nach Anspruch 1, 2, 3, 4 oder 5 als Wirkstoff im Gemisch mit einem pharmazeutisch verträglichen Träger.

20. Verwendung einer Verbindung nach Anspruch 1, 2, 3, 4 oder 5 zur Herstellung eines Medikamentes zur Behandlung von Infektionen, die mit dem Vorhandensein von dagegen empfindlichen Mikroorganismen einher gehen.

21. Verbindung nach Anspruch 1, 2, 3, 4 oder 5 zur Verwendung als Wachstumsförderer bei Tieren.

22. Verwendung einer Verbindung nach Anspruch 1, 2,3, 4 oder 5 zur Herstellung einer Zusammensetzung zur Verwendung als Wachstumsförderer bei Tieren.

## Revendications

1. Composé de formule générale I: 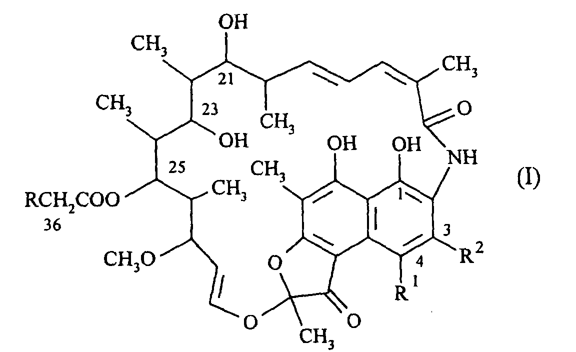 ou leurs dérivés oxydés de formule Ia: 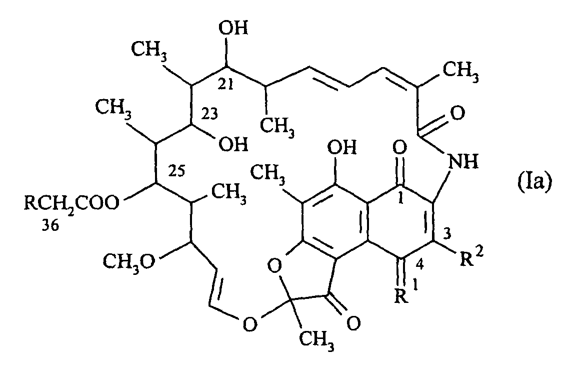 dans lesquelles:
R représente un groupe halogène, hydroxy, thio, alcoxy en C₁-C₄, (alkyl en C₁-C₄)thio, acyloxy en C₁-C₄, (alkyl en C₁-C₄)amino, di(alkyl en C₁-C₄)amino ou un groupe de formule: 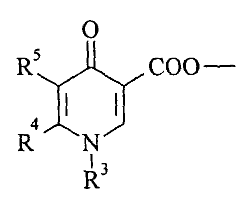 dans laquelle:
R³ représente un groupe alkyle en C₁-C₄ ou cycloalkyle en C₃-C₆;
R⁴ représente un groupe de formule 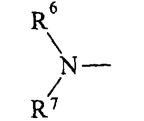 dans laquelle:
R⁶ et R⁷ représentent indépendamment hydrogène ou un groupe alkyle en C₁-C₄ ou
R⁶ et R⁷ forment ensemble avec l'atome d'azote adjacent un noyau hétérocyclique à cinq ou six chaînons, contenant éventuellement un autre hétéroatome choisi parmi l'oxygène, l'azote et le soufre, dans lequel l'un des atomes de carbone ou d'azote du cycle est éventuellement substitué par un reste alkyle en C₁-C₄;
R⁵ est hydrogène ou halogène;
ou R⁴ forme avec R⁵ une chaîne alkylénique bifonctionnelle, contenant éventuellement 1 ou 2 atomes d'azote, ayant les formules suivantes: 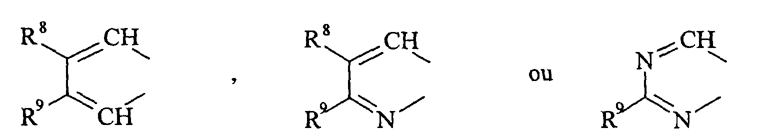 dans lesquelles:
R⁸ représente hydrogène ou halogène;
R⁹ représente alkyle en C₁-C₄ ou un noyau hétérocyclique à six chaînons contenant un ou deux atomes d'azote, dans lequel les atomes de carbone et d'azote du cycle sont éventuellement substitués par un ou deux restes alkyle en C₁-C₄;
R¹ représente hydroxy dans la formule I ou oxygène dans la formule Ia;
R² représente hydrogène, un noyau hétérocyclique à cinq ou six chaînons contenant un ou deux hétéroatomes choisis parmi l'oxygène, l'azote et le soufre, dans lequel un des atomes de carbone ou d'azote du cycle est éventuellement substitué par un reste alkyle en C₁-C₄, ou un groupe de formule:
-CH=N-R¹⁰
dans laquelle R¹⁰ représente un noyau hétérocyclique à six chaînons contenant un ou deux atomes d'azote, dans lequel l'un des atomes de carbone ou d'azote du cycle est éventuellement substitué par un groupe alkyle en C₁-C₄ ou cycloalkyle en C₅-C₆;
ou R¹ et R² pris ensemble forment un groupe de formules =N-(CHR¹¹)-X-, -NH-(CHR¹¹)-X- ou -N=(CR¹¹)-X-,
dans lesquelles X représente un atome de soufre ou un groupe -NH- et
R¹¹ représente un hydrogène, un groupe alkyle en C₁-C₄, (alkyl en C₁-C₄)amino ou di(alkyl en C₁-C₄)amino;
et leurs sels d'addition avec des bases pharmaceutiquement acceptables.

2. Composé selon la revendication 1, dans lequel:
R est halogène, hydroxy, acyloxy en C₁-C₄, alcoxy en C₁-C₄, alkylthio en C₁-C₄, di(alkyl en C₁-C₄)amino ou un groupe de formule: 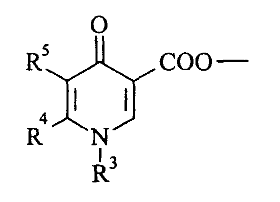 dans laquelle:
R³ représente alkyle en C₁-C₄ ou cycloalkyle en C₃-C₆;
R⁴ représente un groupe de formule 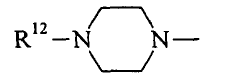 dans laquelle R¹² représente hydrogène ou alkyle en C₁-C₄;
R⁵ est hydrogène ou halogène;
ou R⁴ forme avec R⁵ une chaîne alkylénique bifonctionnelle, contenant éventuellement 1 ou 2 atomes d'azote, des formules suivantes: 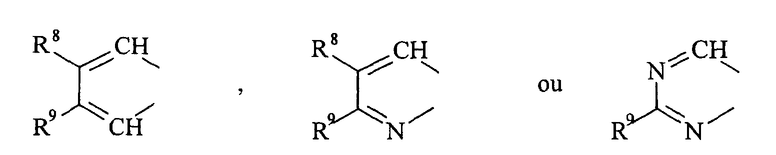
dans lesquelles R⁹ représente alkyle en C₁-C₄, un groupe de formule 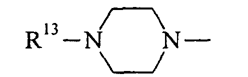
dans laquelle R¹³ est hydrogène ou alkyle en C₁-C₄ ou un groupe de formule 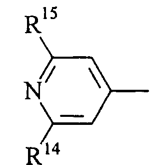
dans laquelle R¹⁴ et R¹⁵ représentent indépendamment hydrogène ou alkyle en C₁-C₄;
R¹ est hydroxy pour la forme réduite ou oxygène pour la forme oxydée;
R² représente hydrogène, un noyau hétérocyclique à six chaînons contenant un ou deux hétéroatomes choisis parmi l'oxygène, l'azote et le soufre, dans lequel un des atomes de carbone ou d'azote du cycle est éventuellement substitué par un reste alkyle en C₁-C₄, ou un groupe de formule: 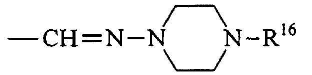
dans laquelle R¹⁶ représente alkyle en C₁-C₄ ou cycloalkyle en C₅-C₆;
ou R¹ et R² pris ensemble forment un groupe de formule -N=CR¹¹-S-, dans laquelle R¹¹ représente hydrogène, alkyle en C₁-C₄ ou di(alkyl en C₁-C₄)amino.

3. Composé selon la revendication 1, dans lequel
R est un groupe fluoro, bromo, chloro, iodo, hydroxy, formyloxy, acétyloxy, thiométhyle, diéthylamino ou un groupe de formule: 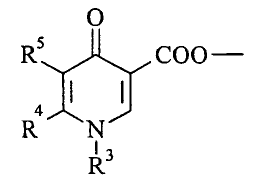 dans laquelle R³ est éthyle ou cyclopropyle, R⁴ est 4-méthyl-1-pipérazinyle et R⁵ est hydrogène;
ou R⁴ forme avec R⁵ une chaîne alkylénique bifonctionnelle, contenant éventuellement 1 ou 2 atomes d'azote, de formules: 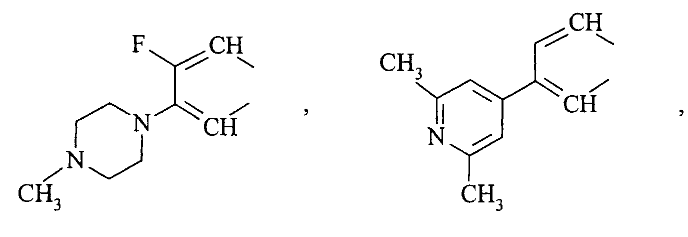 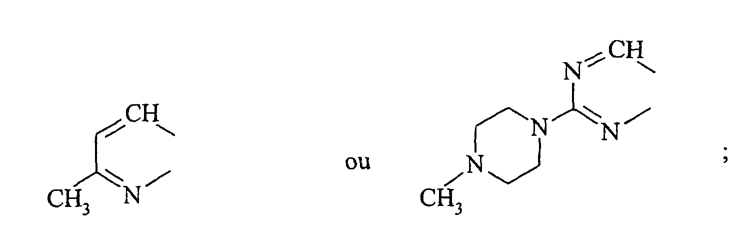
R¹ est hydroxy pour la forme réduite ou oxygène pour la forme oxydée;
R² est hydrogène, 4-morpholinyle, {[(4-méthyl-1-pipérazinyl)imino]-méthyle} ou {[(4-cyclopentyl-1-pipérazinyl)imino]méthyle};
ou R¹ et R² forment ensemble un groupe de formule

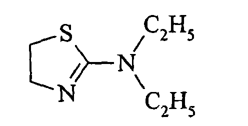

4. Composé selon la revendication 1, dans lequel
R est un groupe bromo, chloro, iodo, hydroxy ou un groupe de formule: 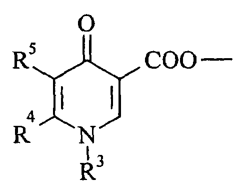 dans laquelle:
R³ est éthyle, R⁴ est 4-méthyl-1-pipérazinyle et R⁵ est hydrogène;
ou R⁴ forme avec R⁵ une chaîne alkylénique bifonctionnelle de formule: 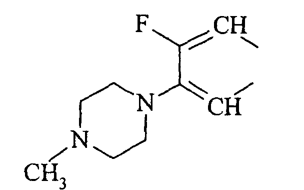
R¹ est hydroxy pour la forme réduite ou oxygène pour la forme oxydée et R² est hydrogène, 4-morpholinyle ou {[(4-méthyl-1-pipérazinyl)imino]méthyle}.

5. Composé selon la revendication 1, 2, 3 ou 4, dans lequel les sels pharmaceutiquement acceptables des composés de formule I sont formés avec un métal alcalin, un métal alcalino-terreux, les (alkyl en C₁-C₄)amines, les (alcanol en C₁-C₄)amines ou les acides aminés basiques.

6. Composé de formule générale 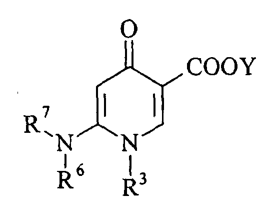 dans laquelle:
Y représente hydrogène ou alkyle en C₁-C₄ et R³, R⁶ et R⁷ sont tels que définis dans la revendication 1,
et ses sels avec des métaux alcalins.

7. Procédé pour la préparation d'un composé de formule générale I, dans laquelle R, R¹ et R² sont tels que définis dans la revendication 1, qui comprend:
a) la réaction d'un composé de formule générale II 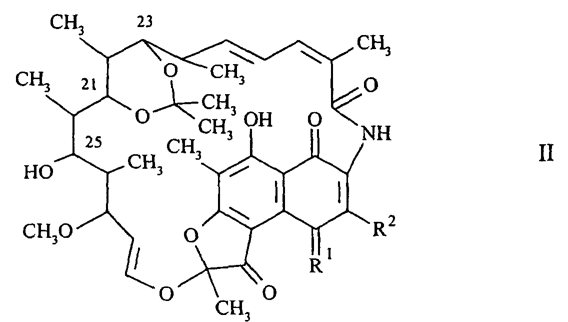 dans laquelle R¹ et R² ont les mêmes significations que dans la revendication 1, à condition que R² ne soit pas un groupe de formule -CH=N-R¹⁰, avec un dérivé d'acide malonique de formule générale III: 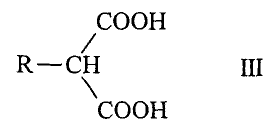 dans laquelle R est tel que défini ci-dessus, en présence d'un agent de condensation et d'un solvant organique inerte;
b) l'élimination de la protection en positions 21 et 23 au moyen d'un clivage acide du reste acétonide en présence d'un solvant organique inerte; et
c) la mise en contact du composé intermédiaire déprotégé obtenu avec un sel ou un oxyde cuivreux ou un de leurs mélanges en présence d'un solvant organique inerte.

8. Procédé selon la revendication 7, dans lequel les solvants organiques inertes des étapes a, b et c sont choisis parmi les alkylamides, les alkylnitriles, les éthers linéaires ou cycliques saturés, les éthers de glycol, les phosphoramides, les sulfoxydes, les solvants chlorés et leurs mélanges.

9. Procédé selon la revendication 7, dans lequel l'agent de condensation de l'étape a est choisi parmi les carboxydiimides, les dialkylaminopyridines, les carbonylimidazoles, la triphénylphosphine, les dithiocarbonates substitués et les diphénylphosphorylazotures.

10. Procédé selon la revendication 7, dans lequel le clivage acide du reste acétonide dans l'étape b est effectué dans des conditions acides douces au moyen d'un acide sulfonique minéral ou organique dilué.

11. Procédé selon la revendication 7, dans lequel l'agent de décarboxylation de l'étape c est choisi parmi Cu₂O, Cu₂S, CuCl, CuBr et Cu₂SO₄ ou un de leurs mélanges.

12. Procédé selon l'une quelconque des revendications 8 à 11, dans lequel la température de réaction de l'étape a est comprise entre 0°C et 35°C, la température de réaction de l'étape b est comprise entre 30°C et 50°C et la réaction de l'étape c est effectuée à une température comprise entre 50°C et 75°C.

13. Procédé pour la préparation d'un composé de formule générale I, dans laquelle R est tel que défini dans la revendication 1, R¹ est oxygène et R² est un groupe de formule -CH=N-R¹⁰, dans laquelle R¹⁰ est tel que défini dans la revendication 1, qui comprend la mise en contact d'un composé de formule générale I, dans laquelle R et R¹ sont tels que définis ci-dessus et R² est hydrogène avec la N-méthylène-tert-butylamine en présence de tert-butylamine et de dioxyde de manganèse et ensuite avec un composé de formule NH₂-R¹⁰, dans laquelle R¹⁰ est tel que défini ci-dessus.

14. Procédé pour la préparation d'un composé de formule générale I, dans laquelle R est iodo et R¹ et R² sont tels que définis dans la revendication 1, qui comprend la mise en contact d'un composé de formule générale I, dans laquelle R est chloro ou bromo et R¹ et R² sont tels que définis dans la revendication 1, avec une solution dans l'acétone d'un iodure de métal alcalin.

15. Procédé pour la préparation d'un composé de formule générale I, dans laquelle R est acyloxy en C₁-C₄, (alkyl en C₁-C₄)amino, di(alkyl en C₁-C₄)amino ou un groupe de formule 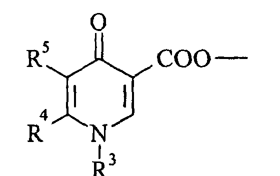 tel que défini dans la revendication 1, R¹ et R² étant tels que définis dans la revendication 1, qui comprend la réaction d'un composé de formule générale I, dans laquelle R est chloro, bromo ou iodo et R¹ et R² sont tels que définis dans la revendication 1, avec respectivement un acylate en C₁-C₄, une (alkyl en C₁-C₄)amine, une di(alkyl en C₁-C₄)amine ou un sel d'un dérivé d'acide 4-oxo-3-pyridinylcarboxylique de formule générale IV 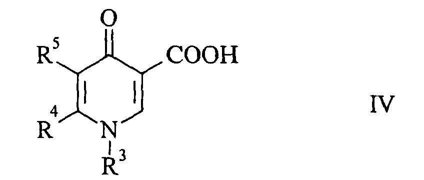 dans laquelle R³, R⁴ et R⁵ sont tels que définis ci-dessus, en présence d'un solvant organique inerte.

16. Procédé selon la revendication 15, dans lequel l'acylate en C₁-C₄ ou le sel du dérivé d'acide 4-oxo-3-pyridinylcarboxylique est un sel d'un métal alcalin ou un sel d'argent.

17. Procédé pour la préparation d'un composé de formule générale I, dans laquelle R est hydroxy et R¹ et R² sont tels que définis dans la revendication 1, qui comprend l'hydrolyse d'un composé de formule générale I, dans laquelle R est formyloxy et R¹ et R² sont tels que définis dans la revendication 1, dans des conditions basiques douces dans une solution hydroalcoolique.

18. Composé selon l'une quelconque des revendications 1, 2, 3, 4 ou 5 pour une utilisation en tant que médicament.

19. Composition pharmaceutique contenant en tant qu'ingrédient actif un composé selon la revendication 1, 2, 3, 4 ou 5 en mélange avec un excipient pharmaceutiquement acceptable.

20. Utilisation d'un composé selon la revendication 1, 2, 3, 4 ou 5 pour la préparation d'un médicament pour le traitement des infections associées à la présence de micro-organismes sensibles à celui-ci.

21. Composé selon la revendication 1, 2, 3, 4 ou 5 pour une utilisation en tant que promoteur de croissance des animaux.

22. Utilisation d'un composé selon la revendication 1, 2, 3, 4 ou 5 pour la préparation d'une composition pour une utilisation en tant que promoteur de croissance des animaux.
